(19) Europäisches Patentamt
European Patent Office
Office européen des brevets

(11) EP 1 970 441 A1

(12) EUROPEAN PATENT APPLICATION

(43) Date of publication:
**17.09.2008 Bulletin 2008/38**

(51) Int Cl.:
***C12N 9/64*** *(2006.01)*

(21) Application number: **07290285.1**

(22) Date of filing: **06.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **BioAlliance Pharma**
**75015 Paris (FR)**

(72) Inventor: **Bouquet, Cécile**
**64310 Orly (FR)**

(74) Representative: **Colombet, Alain André et al**
**Cabinet Lavoix**
**2, place d'Estienne d'Orves**
**75441 Paris Cedex 03 (FR)**

# (54) Plasmid containing a sequence encoding a disintegrin domain of metargidin (RDD)

(57) The invention relates to a pORT plasmid containing a sequence encoding all or part of a disintegrin domain of metargidin (RDD) or a derivative thereof under the control of strong cytomegalovirus promoter, in particular a plasmid having the sequence shown in SEQ ID NO:2.

EP 1 970 441 A1

SEQUENCE LISTING

<110> Bioalliance Pharma

<120> Plasmid containing a sequence encoding a disintegrin domain of metargidin (RDD)

<130> BET 07P0013

<160> 3

<170> PatentIn version 3.3

<210> 1
<211> 276
<212> DNA
<213> Homo sapiens

<220>
<221> misc_feature
<222> (1)..(276)

<400> 1
atggctgctt tctgcggaaa tatgtttgtg gagccgggcg agcagtgtga ctgtggcttc 60

ctggatgact gcgtcgatcc ctgctgtgat tctttgacct gccagctgag gccaggtgca 120

cagtgtgcat ctgacggacc ctgttgtcaa aattgccagc tgcgcccgtc tggctggcag 180

tgtcgtccta ccagagggga ttgtgacttg cctgaattct gcccaggaga cagctcccag 240

tgtcccccctg atgtcagcct aggggatggc gagtaa 276

<210> 2
<211> 2531
<212> DNA
<213> Artificial

<220>
<223> plasmid

<220>
<221> misc_feature
<222> (211)..(231)
<223> lacO3

<220>
<221> misc_feature
<222> (303)..(323)
<223> lacO1

<220>
<221> misc_feature
<222> (423)..(1010)
<223> CMV-intronA promoter

<220>
<221> misc_feature

<222> (1108)..(1167)
<223> secretion signal of human urokinase

<220>
<221> misc_feature
<222> (1168)..(1443)
<223> human RDD gene

<220>
<221> misc_feature
<222> (1481)..(1705)
<223> BGH (bovin growth hormone) polyadenylation signal

<220>
<221> misc_feature
<222> (1858)..(2454)
<223> pMB1 Ori

<400> 2
```
tgattctgtg gataaccgta ttaccgcctt tgagtgagct gataccgctc gccgcagccg      60

aacgaccgag cgcagcgagt cagtgagcga ggaagcggaa gagcgcccaa tacgcaaacc     120

gcctctcccc gcgcgttggc cgattcatta atgcagggcc ggcccggcct aggtaccccc     180

tggcacgaca ggtttcccga ctggaaagcg ggcagtgagc gcaacgcaat taatgtgagt     240

tagctcactc attaggcacc ccaggcttta cactttatgc ttccggctcg tatgttgtgt     300

ggaattgtga gcggataaca atttcacaca ggaaacagct atgaccatga ttacgccaag     360

cgcgcaatta accctcacta aagggaacaa aagctggagc gatgtacggg ccagatatac     420

gcgttgacat tgattattga ctagttatta atagtaatca attacggggt cattagttca     480

tagcccatat atggagttcc gcgttacata acttacggta aatggcccgc ctggctgacc     540

gcccaacgac ccccgcccat tgacgtcaat aatgacgtat gttcccatag taacgccaat     600

agggactttc cattgacgtc aatgggtgga gtatttacgg taaactgccc acttggcagt     660

acatcaagtg tatcatatgc caagtacgcc ccctattgac gtcaatgacg gtaaatggcc     720

cgcctggcat tatgcccagt acatgacctt atgggacttt cctacttggc agtacatcta     780

cgtattagtc atcgctatta ccatggtgat gcggttttgg cagtacatca atgggcgtgg     840

atagcggttt gactcacggg gatttccaag tctccacccc attgacgtca atgggagttt     900

gttttggcac caaaatcaac gggactttcc aaaatgtcgt aacaactccg ccccattgac     960

gcaaatgggc ggtaggcgtg tacggtggga ggtctatata agcagagctc tctggctaac    1020

tagagaaccc actgcttact ggcttatcga aattaatacg actcactata gggagaccca    1080

agctggctag cgtttaaact taagcttatg agagccctgc tggcgcgcct gcttctctgc    1140

gtcctggtcg tgagcgactc caaaggcatg gctgctttct gcggaaatat gtttgtggag    1200

ccgggcgagc agtgtgactg tggcttcctg gatgactgcg tcgatccctg ctgtgattct    1260
```

ttgacctgcc agctgaggcc aggtgcacag tgtgcatctg acggaccctg ttgtcaaaat 1320

tgccagctgc gcccgtctgg ctggcagtgt cgtcctacca gaggggattg tgacttgcct 1380

gaattctgcc caggagacag ctcccagtgt ccccctgatg tcagcctagg ggatggcgag 1440

taatctagag ggcccgttta aacccgctga tcagcctcga ctgtgccttc tagttgccag 1500

ccatctgttg tttgcccctc ccccgtgcct tccttgaccc tggaaggtgc cactcccact 1560

gtcctttcct aataaaatga ggaaattgca tcgcattgtc tgagtaggtg tcattctatt 1620

ctggggggtg gggtggggca ggacagcaag ggggaggatt gggaagacaa tagcaggcat 1680

gctggggatg cggtgggctc tatggcttct gaggcggaaa gaaccaggac ctcgaggggg 1740

ggcccggtac ccaattcgcc ctatagtgag tcgtattacg cgcgctcact ggccgtcgtt 1800

ttacaacgtg agttttcgtt ccactgagcg tcagaccccg tagaaaagat caaaggatct 1860

tcttgagatc cttttttct gcgcgtaatc tgctgcttgc aaacaaaaaa accaccgcta 1920

ccagcggtgg tttgtttgcc ggatcaagag ctaccaactc tttttccgaa ggtaactggc 1980

ttcagcagag cgcagatacc aaatactgtt cttctagtgt agccgtagtt aggccaccac 2040

ttcaagaact ctgtagcacc gcctacatac ctcgctctgc taatcctgtt accagtggct 2100

gctgccagtg gcgataagtc gtgtcttacc gggttggact caagacgata gttaccggat 2160

aaggcgcagc ggtcgggctg aacggggggt tcgtgcacac agcccagctt ggagcgaacg 2220

acctacaccg aactgagata cctacagcgt gagctatgag aaagcgccac gcttcccgaa 2280

gggagaaagg cggacaggta tccggtaagc ggcagggtcg gaacaggaga gcgcacgagg 2340

gagcttccag ggggaaacgc ctggtatctt tatagtcctg tcgggtttcg ccacctctga 2400

cttgagcgtc gatttttgtg atgctcgtca ggggggcgga gcctatggaa aaacgccagc 2460

aacgcggcct ttttacggtt cctggccttt tgctggcctt ttgctcacat gttctttcct 2520

gcgttatccc c 2531

<210> 3
<211> 348
<212> DNA
<213> Artificial

<220>
<223> expression cassette

<400> 3
aagcttatga gagccctgct ggcgcgcctg cttctctgcg tcctggtcgt gagcgactcc 60

aaaggcatgg ctgctttctg cggaaatatg tttgtggagc cgggcgagca gtgtgactgt 120

ggcttcctgg atgactgcgt cgatccctgc tgtgattctt tgacctgcca gctgaggcca 180

ggtgcacagt gtgcatctga cggaccctgt tgtcaaaatt gccagctgcg cccgtctggc 240

```
tggcagtgtc gtcctaccag aggggattgt gacttgcctg aattctgccc aggagacagc    300
tcccagtgtc cccctgatgt cagcctaggg gatggcgagt aatctaga                 348
```

## Description

The invention relates to a pORT plasmid containing a sequence encoding all or part of a disintegrin domain of metargidin (RDD) or a derivative thereof under the control of strong cytomegalovirus promoter, in particular a plasmid having the sequence shown in SEQ ID NO:2. Said plasmid has antitumoral activity *in vivo* and thus provides a promising therapeutic agent for the treatment of cancer.

Angiogenesis, the development of new capillaries from preexisting blood vessels, is essential for the growth and progression of primary solid tumors. A strategy aimed at inhibiting such neovascularization within tumors has thus been proposed (Folkman, Nat Med 1995;1:27-31). Anti-angiogenic therapy presents at least two obvious advantages to contain cancer: (a), application to a vast variety of cancers because angiogenesis is a phenomenon common to all malignancies; and (b), restriction and regression of tumor neovascularization should prevent the passage of tumor cells into the circulation and, therefore, lower the metastatic risk.

The family of adamalysin proteins, also referred to as A Disintegrin And Metalloproteinase Proteins (ADAMs), contains a disintegrin domain located on the metalloproteinase domain. The disintegrin region contains an integrin-binding sequence that interacts with integrins and may mediate cell-cell interactions (Wolfsberg, J Cell Biol 1995;131: 275-8). Metargidin (metalloprotease-RGD-disintegrin protein), also called human ADAM-15, is a transmembrane adamalysin expressed by smooth muscle cells, mesangial cells, and at a much higher level, activated angiogenic endothelial cells (Krätzschmar et al., J Biol Chem 1996;271:4593-6; Ham et al., Exp Cell Res 2002;279:239-47; Herren et al., FASEB J 1997;11:173-80; Martin et al., J Biol Chem 2002;277:33683-9).

AMEP (for Antiangiogenic Metargidin Peptide) is the recombinant disintegrin domain (RDD) of metargidin. The AMEP peptide binds integrins, such as $\alpha 5\beta 1$ and $\alpha v\beta 3$ (Zhang et al., J Biol Chem 1998;273:7345-50; Nath et al., J Cell Sci 1999;112:579-87), via the RGDC integrin-binding sequence, suggesting that the functions of these integrins and metargidin may be mutually dependent. The hypothesis that AMEP, expressed as soluble recombinant protein, might prevent this interaction led to explore the action of RDD on angiogenesis and tumor growth.

RDD was previously demonstrated to inhibit endothelial cell proliferation, migration, invasion and organization in capillary tube like structures *in vitro.* The RDD cDNA, inserted in plasmids, was electrotransferred into mouse skeletal muscles in a tetracycline-inducible system. RDD production *in vivo* inhibited s.c. human mammary MDA-MB-231 tumor growth by 78% at day 14 after transgene electrotransfer into muscles and induction by doxycycline. This antitumor effect was associated with significantly less tumor vascularization. In addition, in the presence of RDD, B16F10 melanoma metastatis was inhibited by 74% in mouse lungs after 1 week of treatment (Trochon-Joseph et al., Cancer Res 2004; 64:2062-2069).

However, this tetracycline-inducible system requires co-transfection of three plasmids (pBi-RDD plasmid as well as Tet-On and Tet-tTS vectors) and associated administration of doxycylin. Hence, a simpler and efficient method of expressing RDD cDNA *in vivo* was thought for further development.

An initial attempt was made with a pVAX plasmid (Invitrogen, V260-20) containing the human cytomegalovirus (CMV) promoter driving a constitutive expression of the transgene, and the kanamycine resistance gene. However, this vector did not achieve fully satisfactory results as regards transgene expression and *in vitro* antiproliferative activity.

The RDD cDNA was further cloned in a plasmid, devoid of any antibiotic resistance gene, dedicated to clinical application in humans, provided by Cobra Biomanufacturing : the pORT-RDD plasmid. This new plasmid is also characterized by : a strong constitutive promoter (CMV-intronA), a human secretion signal (human urokinase uPA signal), a reduced number of immunostimulatory CpG sequences (150 in pORT-RDD), a small size and high copy number. The pORT-RDD vector was unexpectedly found to achieve high level of expression of RDD transgene *in vitro,* associated with a strong antiproliferative activity. Further experiments have been performed *in vivo* which demonstrated antitumoral and antimetastatic activity on animal model of melanoma.

Accordingly, the pORT-RDD vector provides a promising therapeutic for antiangiogenic treatment of cancer and metastases. Since it is devoid of any antibiotic resistance gene, this vector is also particularly appropriate in view of the future guidelines (Guideline EMEA CPMP/BWP/3088/99 ; Directive 2001/18/CE) relating to clinical aspects of gene transfer medicinal products.

### *Definitions*

"Metargidin" denotes the human metalloprotease-RGD-disintegrin protein which also called MDC-15 or ADAM-15. Cloning of metargidin was described by Krätzschmar et al. (J Biol Chem 1996;271:4593-6).

"RDD" denotes the disintegrin domain of human metargidin. The cDNA sequence of RDD is shown in the annexed sequence listing as SEQ ID NO:1. "AMEP" or "Antiangiogenic Metargidin Peptide" denotes recombinant RDD peptide.

The term "derivatives" means a variant of the cDNA sequence of RDD in which one or more nucleotides are substituted, added or deleted from the above-mentioned sequence, as long as it retains a biological activity of wild-type RDD, e.g. inhibition of endothelial cell proliferation and/or inhibition of angiogenesis *in vitro* or *in vivo.* It is preferred that such a variant has a sequence with an identity of at least 80%, preferably 90% or more, more preferably 95% or more with the above-mentioned cDNA sequence.

The term "peptide" is indifferently meant for a dipeptide, an oligopeptide, or a polypeptide, i.e. a polymer in which the monomers are amino acid residues joined together through amide bonds, whatever its length.

"Plasmids" are extrachromosomal double-stranded DNA (dsDNA) molecules which are typically capable of autonomous replication within their hosts, for instance bacteria. As used herein, the terms "vector" and "plasmid" indifferently denote the vehicle by which RDD cDNA sequence can be introduced into a host cell, so as to transform the host and promote expression (e.g. transcription and translation) of the RDD sequence.

A "pORT plasmid" denotes a plasmid comprising an operator liable to binding by a repressor expressed in trans. Said operator may be for instance lac operator, A operator, trp operator, gal operator, ara operator, Arg operator and Tet operator. pORT plasmids have been described in Williams et al., Nucleic Acids Res. 1998 May 1;26(9):2120-4 ; Cranenburgh et al., Nucleic Acids Res. 2001 Mar 1;29(5):E26 ; Cranenburgh et al., J Mol Microbiol Biotechnol. 2004;7 (4):197-203 and in the international patent application WO 9709435.

*pORT-ROD plasmid and production thereof*

The ORT® technology employs a plasmid-mediated repressor titration to activate a host selectable marker, removing the requirement for a plasmid-borne marker gene. As an example, a strain of bacteria can be engineered that contains an essential gene such as *dapD* under transcriptional control of the lac operator/promoter (*lacO*/*P*). In the absence of an inducer such as lactose, this strain cannot grow due to the repression of *dapD* expression by the *LacI* repressor protein binding to *lacO*/*P.* Transformation with a high copy number plasmid containing the lac operator (*lacO*) effectively induces *dapD* expression by titrating *LacI* from the operator. Regulation of the essential gene ensures the growth of bacteria and maintenance of recombinant plasmids containing *lacO* and an origin of replication (Williams et al, Nucleic Acids Res. 1998 May 1;26(9):2120-4 ; Cranenburgh et al., Nucleic Acids Res. 2001 Mar 1;29(5):E26 ; Cranenburgh et al., J Mol Microbiol Biotechnol. 2004;7(4):197-203).

An expression plasmid, pORT-RDD, has been designed which encodes the RDD gene. More specifically, an expression cassette containing the RDD gene driven by human urokinase secretion signal was inserted into a pORT1 aCMV, i.e. a ORT® plasmid containing a strong eukaryotic constitutive promoter (CMV-intronA). The RDD expression cassette is thus under the control of the strong cytomegalovirus (CMV) promoter and placed upstream of the bovine growth hormone (bGH) polyadenylation signal. The sequence of pORT-RDD plasmid is shown in SEQ ID NO:2.

Thus, the invention relates to a pORT plasmid containing a sequence encoding all or part of a disintegrin domain of a metargidin, or a derivative thereof, wherein said sequence is inserted under the control of the strong cytomegalovirus (CMV) promoter.

In particular, the plasmid containing a sequence encoding disintegrin domain of metargidin (RDD), may have the sequence shown in SEQ ID NO:2.

Said plasmid, also called pORT-RDD plasmid, may be produced according to any suitable method, in particular recombinant DNA technology. For instance, the pORT-RDD plasmid may be amplified in a suitable host cell, such as a bacterial host cell, in particular a *Escherichia coli* bacterium, more specifically a *Escherichia coli* engineered to contain an essential chromosomal gene, such as *dapD,* under transcriptional control of the lac operator/promoter (lacO/P).

Furthermore, the pORT-RDD plasmid is an expression plasmid which makes it possible to express the RDD peptide when transfected into a suitable host cell, for instance a mammalian host cell, in particular a tumor cell.

Thus, the invention further provides a host cell transformed with pORT-RDD plasmid. The term "host cell" means any cell of any organism that is selected, modified, transformed, grown, or used or manipulated in any way, for the production of a substance by the cell, for example the amplification by the cell of pORT-RDD plasmid, or for the expression of RDD peptide by the cell.

*Ex vivo* or *in vivo* introduction of pORT-RDD plasmid in the host cell may be performed by any standard method well known by one skilled in the art, e.g. transfection, electroporation, microinjection, transduction, cell fusion, DEAE dextran, calcium phosphate precipitation, use of a gene gun, or lipofection.

The invention further provides a method of : a) producing pORT-RDD plasmid, which method comprises the steps consisting of culturing a host cell transformed with said pORT-RDD plasmid, and b) recovering the pORT-RDD plasmid from the cultured host cells.

For producing and amplifying pORT-RDD plasmid, use may be made in particular of *Escherichia coli* DH1-strains as described by Cranenburgh et al. (Nucleic Acids Res. 2001 Mar 1;29(5):E26), i.e. strains that contain the *dapD* chromosomal gene under the control of the lac operator/promoter. These strains are available by Cobra Biomanufacturing. Transformed host cells may be readily isolated and propagated because, unless cultured in a medium supplemented with IPTG (which induces expression of *dapD*) or DAP, only transformants can grow on any medium by repressor titration selection.

pORT-RDD plasmid may be readily produced by the one skilled in the art, for instance by inoculating an appropriate culture medium, such as LB medium (i.e. peptone 10 g/l, yeast extract 5 g/l, NaCl 5 g/l), with a transformed ORT-strain and culturing. On a laboratory scale, this may be performed in conventional laboratory flask by incubating at 37°C, preferably with shaking (for instance 200 rpm).

pORT-RDD plasmid may also be produced on a larger scale in a fermenter. Advantageously, a fermentation may be carried out according to the protocol described by Varley et al. (Bioseparation. 1999;8(1-5):209-17), or according to a adaptation thereof. A suitable culture medium for performing fermentation contains $KH_2PO_4$ 3 g/l, $Na_2HPO_4$ 6 g/l, NaCl 0.5 g/l, polypropylene glycol MW 2025g 0.2%, trace element solution, $CaCl_2.2H_2O$ 0.03 g/l, $FeSO_4.7H_2O$ 0.04 g/l, citric acid 0.02 g/l and, $MgSO_4$ 0.5 g/l, vitamin solution, tetracycline 12 mg/l. The medium may further comprise peptone 2 g/l, yeast extract 20 g/l, $(NH_4)_2S0_4$ 10 g/l, glycerol 2.8 %. Alternatively peptone, yeast extract, $(NH_4)_2SO_4$ and glycerol may be fed continuously into the fermentation vessel. Fermentation is preferably carried out at 37°C, pH 6.8 with dissolved oxygen setpoint 50% of air saturation.

Culture harvests are generally centrifuged to pellet the cells. Cell pellets may then be frozen and stored at -70°C before purification.

The choice of an appropriate method of purifying the pORT-RDD plasmid is within the ordinary skills of the skilled person.

Preferably purification may be achieved by the steps of : (a) alkaline lysis of cultured host cells followed by a neutralization; (b) mesh bag filtration; (c) anion exchange chromatography; (d) concentration; (e) ion-pair reverse phase and size exclusion chromatographies, and (f) filtration.

Accordingly, the invention further provides a method of producing pORT-RDD plasmid which comprises the steps consisting of:

a) culturing a host cell transformed with the pORT-RDD plasmid;
b) recovering the pORT-RDD plasmid from the cultured host cells; and
c) purifying said pORT-RDD plasmid by (i) alkaline lysis of cultured host cells and neutralization; (ii) mesh bag filtration; (iii) anion exchange chromatography; (iv) concentration/diafiltration; (v) ion-pair reverse phase and size exclusion chromatographies, and (vi) filtration.

The invention also relates to a method of purifying pORT-RDD plasmid from a transformed host cell, which method comprises the steps of : (a) alkaline lysis of host cells followed by a neutralization; (b) mesh bag filtration; (c) anion exchange chromatography; (d) concentration; (e) ion-pair reverse phase and size exclusion chromatographies, and (f) filtration.

A method of production and/or purification of pORT-RDD plasmid is further detailed in the following Example 1.

Where the host cell is a mammalian cell, the invention further provides a method of *in vitro* or *in vivo* expressing RDD peptide in a cell, which comprises transforming said cell with pORT-RDD plasmid, whereby the RDD peptide (or AMEP, since it is recombinant RDD peptide) is expressed in the cell. The mammalian cell is in particular a tumor cell.

Expression of the RDD peptide may found application in situations where angiogenic activity is to be inhibited, for instance in cancer. Therapeutic applications are further detailed below.

*Pharmaceutical compositions and therapeutic applications*

The invention further provides the use of the pORT-RDD plasmid for the preparation of a medicament intended for the treatment of tumor.

It also provided a method of treating tumor which comprises administering a subject in need thereof with a therapeutically effective amount of pORT-RDD plasmid.

In the context of the invention, the term "treating" or "treatment" means reversing, alleviating, inhibiting the progress of, or preventing the disorder or condition to which such term applies, or one or more symptoms of such disorder or condition.

"Therapeutically effective amounts" are those amounts effective to produce beneficial results, particularly with respect to cancer treatment, in the recipient subject. Such amounts may be initially determined by reviewing the published literature, by conducting *in vitro* tests or by conducting metabolic studies in healthy experimental animals.

As used herein, the term "subject" denotes a mammal, such as a rodent, a feline, a canine, and a primate. Preferably a subject according to the invention is a human.

Since RDD peptide has anti-angiogenic activity, therapy with pORT-RDD plasmid may find application in vast variety of cancers and may prevent the passage of tumor cells into the circulation, i.e. prevent the occurrence and/or development of metastases.

According to the invention, the tumor may be any tumor, e.g. a primary or metastatic tumor, a solid tumor or soft tissue tumor, or a hematologic malignancy. Indeed, increasing evidence indicates that angiogenesis also plays an important role in hematologic malignancies (Dong et al., Crit Rev Oncol Hematol. 2006 Dec 21). Examples of solid or soft tumor include bladder, breast, bone, brain, cervical, colorectal, endometrial, kidney, liver, lung, nervous system, ovarian, prostate, testicular, thyroid, uterus, and skin cancer, especially melanoma. Hematologic malignancies include for instance acute or chronic leukaemias, such as acute myeloid leukaemia, acute lymphoblastic leukaemia, chronic myeloid leukaemia, or chronic lymphocytic leukaemia; lymphoma; multiple myeloma; and myelodysplastic syndromes.

According to an embodiment, the medicament or the method of treatment according to the invention is intended for the prevention and/or treatment of a metastatic tumor.

While it is possible for the pORT-RDD plasmid to be administered alone, it is preferable to present pORT-RDD as pharmaceutical composition.

Hence, the invention further provides a pharmaceutical composition comprising the pORT-RDD plasmid together with a pharmaceutically acceptable carrier.

As used herein, the term "pharmaceutically acceptable" and grammatical variations thereof, as they refer to compositions, vehicles, carriers, diluents and reagents, are used interchangeably and represent that the materials are capable of administration to or upon a subject without the production of undesirable physiological effects such as nausea, dizziness, gastric upset and the like. Suitable pharmaceutically acceptable carriers known in the art include, but are not limited to, sterile water, saline, glucose, dextrose, or buffered solutions. Carriers may include auxiliary agents including, but not limited to, diluents, stabilizers (i.e., sugars and amino acids), preservatives, wetting agents, emulsifying agents, pH buffering agents, viscosity enhancing additives, colors and the like.

The pharmaceutical composition comprising pORT-RDD plasmid can be administered intravenously, intradermally, intraarterially, intraperitoneally, intralesionally, intracranially, intraarticularly, intraprostaticaly, intrapleurally, intratracheally, intranasally, intravitreally, intravaginally, rectally, topically, intratumorally, intramuscularly, intraperitoneall y, subcutaneously, intravesicularly, mucosally, intrapericardially, orally, topically, locally and/or using aerosol, injection, infusion, continuous infusion, localized perfusion bathing target cells directly or via a catheter and/or lavage. For example, a pORT-RDD composition of the present invention may be formulated for parenteral administration, e.g., formulated for injection via the intravenous, intramuscular or subcutaneous routes, though other routes such aerosol administration may be used. The preparation of an aqueous composition that contains the pORT-RDD plasmid of the present invention and/or and additional agent as an active ingredient will be known to those of skill in the art in light of the present disclosure, as exemplified by Remington's Pharmaceutical Sciences, 16th Ed. Mack Publishing Company, 1980.

Typically such compositions are prepared as injectables either as liquid solutions or suspensions; solid forms suitable for preparing solutions or suspensions upon the addition of a liquid prior to injection can also be prepared. The preparation can also be emulsified. In particular, the pharmaceutical compositions may be formulated in solid dosage form, for example capsules, tablets, pills, powders, dragees or granules. Sterile injectable solutions are prepared by incorporating the plasmid in the required amount in the appropriate solvent with various of the other ingredients enumerated above, as required, followed by filtered sterilization. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area. The compositions will be sterile, be fluid to the extent that easy syringability exists, stable under the conditions of manufacture and storage, and preserved against the contaminating action of microorganisms. Examples of appropriate solvents usable for preparing injectable compositions include a TENaCl solution consisting of 10mM Tris 1 mM EDTA, 150mM NaCl, pH7.5, or physiologic serum (NaCl 0.9%).

The formulations can be prepared in unit dosage form by any of the methods well known in the art of pharmacy. Such methods include the step of bringing into association the pORT-RDD plasmid with the carrier which constitutes one or more accessory ingredients. In general the formulations are prepared by uniformly and intimately bringing into association the pORT-RDD plasmid with liquid carriers or finely divided solid carriers or both, and then, if necessary, shaping the product.

The actual dosage amount of a pORT-RDD plasmid composition of the present invention (and/or an additional agent) for administration to a subject can be determined by physical and physiological factors such as body weight, tumor volume, severity of condition, idiopathy of the subject and on the route of administration. With these considerations in mind, the dosage of a lipid composition for a particular subject and/or course of treatment can readily be determined.

Treatment may vary depending upon the subject treated and the particular mode of administration. For example, in the invention the dose range of pORT-RDD plasmid may be about 0.05 mg/kg body weight to about 500 mg/kg body weight. The term "body weight" is applicable when an animal is being treated. When isolated cells are being treated, "body weight" as used herein should read to mean "total cell weight". The term "total weight" may be used to apply to both isolated cell and animal treatment. However, those of skill will recognize the utility of a variety of dosage range, for example, 0.05 mg/kg body weight to 300 mg/kg body weight, 0.1 mg/kg body weight to 100 mg/kg body weight, 0.2 mg/kg body weight to 50 mg/kg body weighty, or 0.5 mg/kg body weight to 10 mg/kg body weight. A preferred dosage regimen may be between 0.1 and 1 mg/kg body. Of course, all of these dosages are exemplary, and any dosage in-between these points is also expected to be of use in the invention. The specific dose level for any particular subject will depend upon a variety of factors including the body weight, general health, sex, diet, time and route of administration, rates of absorption and excretion, combination with other drugs and the severity of the particular cancer being treated.

Any suitable mode of administration of the pORT-RDD plasmid may be used by the one skilled in the art. Such methods include, but are not limited to direct delivery of the plasmid by: injection, microinjection, electroporation, electrotransfer, jet-injection, calcium phosphate precipitation, using DEAE-dextran followed by polyethylene glycol, direct sonic loading, liposome mediated transfection, receptor-mediated transfection, microprojectile bombardment, agitation with silicon carbide fibers, PEG-mediated transformation of protoplasts, desiccation/inhibition-mediated DNA uptake, or any combination of such methods. Preferably pORT-RDD plasmid is administered by electrotransfer, as further described below.

*Electrotransfer protocol*

The invention further provides the use of the pORT-RDD plasmid for the preparation of a medicament intended to be electrotransferred *in vivo* into tumor cells.

The plasmid to be administered may be electrotransferred according to any suitable method known in the art.

Advantageously, electrotransfer may be performed according to the protocol described in the international patent application PCT/IB2006/002401.

According to this protocol, the plasmid or medicament is contacted with tumor cells and the tumor is electrically stimulated as follows:

- first with at least one pulse of a High Voltage (HV) field strength of between 200 and 2000 volts/cm
- second with a single pulse of Low Voltage (LV) field strength of between 50 and 200 volts/cm and of duration of between 300 and 2000 ms.

The plasmid may be contacted with the tumor cells from a few seconds to 10 minutes, e.g. from 30 s and 5 minutes, before applying the HV pulse(s).

The plasmid may be brought into contact through direct intratumoral injection, through systemic administration (e.g. intravenous or intra-arterial route) or by topical or subcutaneous administration. Intramuscular injection of the plasmid may be used to contact the plasmid with tumor cells.

In particular, tumor tissue may be electrically stimulated first with at least one pulse of a HV field strength of between 400 and 2000 volts/cm, preferably between 600 and 2000, preferably of between 800 and 1600 volts/cm, more preferably of between 900 and 1200, typically about 1000 volts/cm. The HV pulse may have a duration of between 10 and 1000 $\mu$s, preferably of between 50 and 200 $\mu$s, typically about 100 $\mu$s.

There may be several HV pulses, i.e. from 2 to 10 HV pulses having the specifications disclosed therein. It is more convenient in this case to have identical HV pulses. The frequency of HV pulses may be 1 Hz. However, a single HV pulse as disclosed above is sufficient to permeabilize the cell membrane. Therefore, in the preferred embodiment, use is made of a single HV pulse.

Where there is a single HV pulse, it is preferably a squared pulse. In case of several HV pulses, use can be made of unipolar or bipolar pulses, or of pulses having different directions and/or polarities, preferably of the squared type.

According to an embodiment, the at least one pulse of a HV field strength consists of one pulse HV = 1500 V/cm, 100 $\mu$s, 1 Hz.

The HV and LV pulses may be separated by lag and this lag can advantageously be between 300 ms and 3000 s, preferably between 500 ms and 1000 s, typically about 1000 ms. In a particular embodiment, there is no lag or only a short one, say less than 300 ms, and the HV pulse has a field strength of between 300 and 1000 volts/cm, preferably of between 400 and 800 volts/cm.

The single LV pulse may have a field strength of between 100 and 200 volts/cm, preferably of between 120 and 160 volts/cm, typically about 140 volts/cm. The single LV pulse may have a duration of between 300 and 800 ms, preferably of between 350 and 600 ms, typically about 400 ms.

The LV pulse may be of the same polarity than the HV pulse, or may have a polarity opposed to that of the HV pulse. Preferably, the single LV pulse is a squared pulse. It can also be trapezoidal, or discontinuous.

According to an embodiment, the LV pulse has field strength of 140 V/cm and lasts 400 ms.

Preferably, the following electrotransfer protocol may be used :

- HV = 1300 V/cm, 100 $\mu$s, 1 Hz, 1 pulse
- pause = 1 000 ms
- LV = 140 V/cm, 400 ms, 1 pulse.

The invention will be further illustrated in view of the following figures and examples.

## FIGURES

Figure 1 is the map of pORT1aCMV plasmid.
Figure 2 is the map of pORT-ROD plasmid.
Figure 3 is the map of pVAX1 plasmid.
Figure 4 is a representation of tumor growth of B16F10 subcutaneous tumors during 14 days, after intratumoral electrotransfer of pORT-based vectors. 50 µg of plasmid (in 50 µl 10mM Tris, 1 mM EDTA, 0.9% NaCl, pH 7.5) were injected and electric pulses were applied, when tumor volumes were > 30 $mm^3$ (day 0). Data represent the tumor volume (mean ± SD) for each group (at day 0, pORT1aCMV n= 10 (one natural death during experiment) and pORT-RDD n= 11 (two natural deaths during experiment) ; at day 14, n=9 for each group). Stars indicate statistical significance.
Figure 5 is a representation of tumor growth of B16F10 subcutaneous tumors during the first 7 days, after intratumoral electrotransfer of pORT-based vectors. Data represent the tumor volume (mean ± SD) for each group. Stars indicate statistical significance.
Figure 6 displays analysis of tumor volume monitoring in $mm^3$ for small B16F10 subcutaneous tumors treated by intratumoral electrotransfer of pORT-based vectors. Tumor volumes were comprised between 33.51 and 65.45 $mm^3$ at day 0. Data represent the tumor volume (mean ± SD) for each group. Dead mice were excluded of this graph : pORT1aCMV n=6 and pORT-RDD n=7. There is no statistical significance.
Figure 7 displays analysis of tumor monitoring expressed in ratio V/VO for small B16F10 subcutaneous tumors treated by intratumoral electrotransfer of pORT-based vectors. Tumor volumes were comprised between 33.51 and 65.45 $mm^3$ at day 0. V/VO represents the ration between tumor volume at day 0 and tumor volume at day D. Data represent the tumor ratio (mean ± SD) for each group. Dead mice were excluded of this graph : pORT1aCMV n=6 and pORT-RDD n=7. Stars indicate statistical significance.
Figure 8 displays analysis of tumor growth of B16F10 subcutaneous tumors after intratumoral electrotransfer of increasing doses of pORT-RDD plasmid. Increasing doses of pORT-RDD plasmid were injected into pre-established B16F10 subcutaneous tumors and electric pulses were applied, when tumor volumes comprised between 30 and 50 $mm^3$ (day 0). Data represent the tumor volume (mean ± SD) for each group.

## EXAMPLES

### Example 1: production of pORT-RDD plasmid

• Plasmids

Cobra BioManufacturing has generated an expression plasmid, pORT-RDD, encoding RDD gene translationally fused downstream of the signal peptide of human urokinase. The coding sequence is under the control of the strong eukaryotic cytomegalovirus (CMV) promoter and placed upstream of the bovine growth hormone (bGH) polyadenylation signal.
pORT-RDD has been designed by inserting an gene cassette containing RDD gene driven by human urokinase secretion signal into Cobra BioManufacturing's pORT1aCMV plasmid (map in Figure 1).
The sequence of the gene cassette was as follows:

**AAGCTT**ATGAGAGCCCTGCTGGCGCGCCTGCTTCTCTGCGTCCTGGTCGTGAGCG
ACTCCAAAGGC*ATGGCTGCTTTCTGCGGAAATATGTTTGTGGAGCCGGGCGAGCA*
*GTGTGACTGTGGCTTCCTGGATGACTGCGTCGATCCCTGCTGTGATTCTTTGACCT*
*GCCAGCTGAGGCCAGGTGCACAGTGTGCATCTGACGGACCCTGTTGTCAAAATTG*
*CCAGCTGCGCCCGTCTGGCTGGCAGTGTCGTCCTACCAGAGGGGATTGTGACTTG*
*CCTGAATTCTGCCCAGGAGACAGCTCCCAGTGTCCCCCTGATGTCAGCCTAGGGG*
*ATGGCGAGTAA***TCTAGA** (348 bp) (SEQ ID NO:3).

*HindIII* and *XbaI* restriction sites are shown in bold, human urokinase secretion signal is underlined, and RDD gene is in italics.

The synthetic gene cassette and pORT1aCMV have been digested with *HindIII* and *XbaI* and the fragments ligated to create the pORT-RDD plasmid.

pORT-RDD vector thus contains the following elements (plasmid maps in Figure 2): Human cytomegalovirus immediate-early (CMV) promoter + intron a, stronger promoter than the classical CMV (Cobra source), bovine growth hormone (bGH) polyadenylation signal, LacO sequences for selection and human RDD gene under the control of the human urokinase secretion signal.

pORT-RDD was then transformed in the host strain DH1-ORT and manufactured by fermentation. The DH1 Lac dapD strain referenced in Cranenburgh et al., 2001 may also be used.

• pORT-RDD production

*Production of research working cell banks*

A transformed glycerol stock of DH1-ORT (Cobra Biomanufacturing, CTL 2006#0492P) was used to inoculate 8 x 5 ml of sterile LB media in 25 ml sterile universals. The 5 ml cultures were incubated in a shaking incubator at 200 rpm and 37°C for approximately 16 hours. 2 OD ml samples were minipreped to confirm the presence of the plasmid. 1 OD ml of a selected culture was then used to inoculate 25 ml of LB media. This was grown at 37°C with shaking at 200 rpm until it reached an optical density (600 nm) of between 0.8-1.5 OD units. The cultures were cryopreserved using 20 % v/v glycerol. This was aliquoted into 1 ml ± 0.2 ml in more than 20 sterile cryovials and stored below -70°C.

*Genetic stability testing*

The structural and segregational stability of the plasmid was evaluated for greater than 40 cell generations. This was performed by inoculating the same number of cells (0.10 OD ml) into fresh LB media (5 ml) and sub-culturing at 24 hour intervals. Cultures were incubated in an orbital shaker at 37°C and 200 rpm. At each 24 hour interval, the optical density (600 nm) was measured and used to calculate the cell doubling time (or generations) elapsed :

$$\text{No. of cell generations} = \frac{\text{Ln (final Optical Density/Optical Density at inoculation)}}{\ln 2}$$

This was repeated until greater than 40 cell generations had been achieved. A known number of cells (2 OD ml) at each sub-culture stage were pelleted by centrifugation and the pDNA extracted using two miniprep methods : the miniprep method devised by Birnboim and Doly (Nucleic Acids Res. 1979 Nov 24;7(6):1513-23) and using the QIAprep Miniprep method and kit (Qiagen™). An equal volume of the isolated pDNA from each sub-culture was then loaded onto 0.8 % agarose gels stained with ethidium bromide and qualitatively visualised for signs of segregational or structural instability.

There was no obvious sign of gross segregational or structural instability by serial subculture and sampling over 44 generations.

*5-litre fermenter evaluation*

The DH1-ORT strain containing pORT-RDD was evaluated at 5 l scale in FT Applikon 5/7 l fermenter vessels. The complex media and the fermentation protocol used are those in Varley et al. (Bioseparation. 1999;8(1-5):209-17), with the following modifications.

The media described by Varley et al. consisted of final concentrations $KH_2PO_4$ 3 g/l, $Na_2HPO_4$ 6 g/l, NaCl 0.5 g/l, polypropylene glycol MW 2025g 0.2%, trace element solution 0.05 % [consisting of $CoCl_2.6H_2O$ 2g/l, $CuCl_2.2H_2O$ 1.9 g/l, $H_3BO_3$ 1.6 g/l, $MnSO_4.H_2O$ 1.6 g/l, $Na_2MoO_4.2H_2O$ 2 g/l, $ZnCl_2.7H_2O$ 2 g/l, $Fe_2(SO_4)_3.xH_2O$ 1g/l, $CaCl_2.2H_2O$ 1 g/l, and citric acid 60 g/l], $CaCl_2.2H_2O$ 0.03 g/l, $FeSO_4.7H_2O$ 0.04 g/l, citric acid 0.02 g/l, $MgSO_4$ 0.5 g/l, vitamin solution 5 ml/l [consisting of biotin 0.6 g/l, folic acid 0.04 g/l, pyridoxine-HCl 1.4 g/l, riboflavin 0.42 g/l, pantothenoic acid 5.4 g/l, and niacin 6.1 g/l], tetracycline 12 mg/l, as well as peptone 2 g/l, yeast extract 20 g/l, $(NH_4)_2SO_4$ 10 g/l, and glycerol 2.8 %.

However, the glycerol, phytone peptone, yeast extract and ammonium sulphate for the linear fed batch fermentation were excluded from the bulk media and fed into the vessel at a constant feed rate of 60, 40 and 30 ml/hour. The feed solution is prepared by mixing 100 g Bacto yeast extract, 10 g Phytone, 176.5 g glycerol, 25 g ammonium sulphate and adding purified water up to 740 ml. The feed solution is further filtered on a 0.2 μm filter.

Samples for analysis were collected throughout the time course of the fermentation. Samples were analysed for optical density (600 nm), segregational or structural instability, and pDNA specific yields.

The inoculum was prepared using 200 μl from the Research Working Cell Bank into 200 ml T-Broth Media (11.8 g/l Phytone Peptone, 23.6 g/l Yeast extract, 2.2 g/l $KH_2PO_4$, 9.4 g/l $K_2HPO_4$, 5.04 g/l Glycerol) in 2 1 baffled Erlenmeyer shake flasks. The inoculums were incubated at 37°C with shaking at 200 rpm for approximately 12 hours. 1200 OD ml were then used to inoculate the 51 vessel.

*Physical parameters*

Dissolved oxygen, pH, agitation speed and temperature were constantly monitored throughout the fermentation time course.

*Harvest*

The vessel was chilled to 10°C using a jacket supplied with mains water. The culture was collected into 1 l centrifuge pots and the cells pelleted by centrifugation at 4500 rpm for 20 min at 4°C in a Sorvall RC 3B Plus centrifuge. The supernatant was decanted and disinfected with 2 % Tego 2000™. The cell pellets were then frozen at <-70°C.

*Analysis*

The optical density (600 nm) was measured throughout the time course of the fermentation in order to obtain the growth kinetics.

A known amount of cells (*i.e.* $OD_{600}$ x ml) (100 OD ml) were pelleted at several time intervals along the time course of the fermentation. These samples were stored at -20°C. The plasmid from these cells was isolated using the Qiagen-500 Maxi-prep™ kits and protocol, (Qiagen™). The concentration of the pDNA was calculated by measuring the OD of the DNA at 260 nm. From this, the specific plasmid yield of the culture was calculated by dividing the pDNA concentration (μg/ml) by the number of OD ml of the sample. This was done for each fermentation and 0.8 % agarose gels stained with ethidium bromide were run for each sample to qualitatively visualise the pDNA.

A feed rate of 40 ml/hour was eventually found optimal for plasmid yields (about 1 μg/OD ml) during fermentation.

• pORT-RDD purification

All buffer salts and reagents were purchased from VWR International (Leicestershire, UK) and were of analytical grade (AnalaR) unless stated otherwise. All buffers and sanitising solutions were made using purified water and water for injection (WFI) grade water (Hyclone, UK). Optical density measurements were carried out using a DU800 spectrophotometer (Beckman Coulter, UK). Centrifugation was carried out using a Sorvall Evolution RC (Kendro, UK). Agarose gel electrophoresis was performed in an EC CSSU1214 Turn & Cast Gel System (VWR International, UK) and using the Electrophoresis Power Supply 600 (Amersham Biosciences, UK). Components of the Qiagen Plasmid Maxi Kit (Qiagen-tip 500, buffers QBT, QC and QF) (Qiagen, UK) were used for the lysis titration procedure.

*Cell lysis and lysate clarification*

21 of cell suspension buffer was added to the frozen cell paste in a 10 l stainless steel vessel (final concentration 120 g/l for batch 1 and 150 g/l for batch 2) and mixed manually with a stainless steel spatula until the suspension appeared to be free of aggregates. The cells were lysed by mixing with 4 l of lysis solution (0.155 M NaOH, 1% SDS at 20-22°C) for 3 minutes for batch 1 and 10 minutes for batch 2. 2 l of neutralisation solution (3M potassium acetate with 10 mM EDTA at pH 5.5) was added to the lysate whilst stirring gently. The precipitated cell debris was then removed using a 500 μm mesh bag filter (L1 NM500BBSS, Plastok, UK) and nominal 50 μm, 5 μm and 0.3 μm depth filters (KR50A05HH1, KR05A05HH1 and KRK3A05TT1, Millipore, UK).

*Ion-exchange chromatography*

A XK 50/30 chromatography column was packed with 354 ml of purified water washed Fractogel EMD TMAE (Merck KGaA, Germany) to a packed bed volume of 264.6 ml resulting in a bed height of 13.5 cm for batch 1. Batch 2 was packed with 370 ml of purified water washed Fractogel EMD TMAE (Merck KGaA, Germany) to a packed bed volume of 254.8 ml resulting in a bed height of 13.0 cm for batch 2. A packing test was performed on the packed media by applying 2.5 ml of 1 % acetone, resulting in an acceptable Asymmetry value of 1.65 and 1.35 for batch 1 and 2, respectively. The media was then sanitised with two column volumes of 0.5 M NaOH with a contact time exceeding 1 hour. Equilibration buffer was then pumped through the column until the pH of the effluent had reached ≤ 8.5. The column was loaded with 6312.5 ml (batch 1) and 6393.3 ml (batch 2) of clarified feedstock, operated at a constant linear flow rate of 100 cm/hour. Equilibration buffer was re-applied to wash out unbound and contaminating material before the pDNA was recovered by applying elution buffer at a flow rate of 100 cm/hour.

*Concentration of post-ion exchange intermediate by tangential flow filtration*

A Millipore LabScale TFF system was used and fitted with a new 50 $cm^2$, 30 kDa regenerated cellulose Pellicon XL membrane (Millipore, UK). The system and membrane were washed using purified water for 10 minutes, then equilibrated using ion exchange elution buffer (2 x 500 ml, 10 minutes). Post ion-exchange pDNA solution was poured into the reservoir. The pump was switched on and the system allowed to operate for 10 minutes before a 15 psi pressure difference across the membrane was applied. The reservoir was continuously filled until all the eluted material had been added and the volume reduced to a final nominal concentration of about 2 mg/ml pDNA. The concentrate was then drained out from the system before a final buffer rinse ensured that the maximum amount of pDNA was recovered from the membrane. The volume of the rinse buffer was calculated to achieve a final pDNA concentration of 1 mg/ml. in between use, the system and membrane were washed with purified water. The system was then rinsed with water and stored in 0.1 M NaOH.

*Ion pair reverse phase chromatography*

A VS60 chromatography column (Millipore, UK) containing about 850 ml of Perfluorosorb-S media was used during this procedure. Sanitisation of the column and media was carried out by pumping two column volumes of 0.5 M NaOH with a contact time exceeding 1 hour. The media was then equilibrated (until pH had reached 7 ±0.2) before use. The column was loaded with 69.0 ml (batch 1) and 200.8 ml of post-ion exchange TFF concentrate and operated at a constant linear flow rate of 70 cm/hour. Two column volumes of Wash Buffer 1 and four column volumes of Wash Buffer 2 were then introduced into the column to remove residual Endotoxin, RNA and unbound material, before Elution Buffer was used to recover the captured pDNA. The eluted peak was collected and analysed using UV spectrophotometry to determine the concentration of pDNA recovered.

*Concentration/diafiltration by tangential flow filtration and product formulation*

The system was prepared as before. The reservoir was continuously filled until all the pDNA solution had been added. Once the pDNA solution had been concentrated to the required concentration, diafiltration was carried out against formulation buffer (10 mM Tris base, 1 mM EDTA, 0.9 % w/v NaCl pH 7.5) until the pH and conductivity of the permeate was equal to the diafiltration buffer. The diafiltered concentrate was then drained out from the system before 2 x buffer rinses ensured that the maximum amount of pDNA was recovered from the membrane. The recovered pDNA aliquots were filtered using 0.2 μm filter and filled into sterile cryovials under aseptic conditions.

The purification process is summarized in Table 1.

Table 1 : purification summary table

| **pORT-RDD** | |
|---|---|
| Cell lysis | |
| Frozen cell paste used | 298.4 g |
| Theoretical pDNA yield | 158.08 mg |
| Cell suspension buffer | 200 ml |
| RNase A concentration used | 0.1 mg/ml |
| NaOH concentration used | 0.155 M |
| Volume of lysis solution used | 4 000 ml |
| Volume of neutralization solution used | 2 000 ml |
| Clarified lysate produced | 5 833.3 ml |
| Anion exchange chromatography | |
| 3 M NaCl solution added | 583.0 ml |

(continued)

| Anion exchange chromatography | |
|---|---|
| TMAE media used | 370 ml |
| Packed bed height | 13.0 cm |
| Cross sectional area | 19.6 $cm^2$ |
| Packed bed volume | 254.8 ml |
| Load volume | 6 398.3 ml |
| Conductivity of load | 71.8 @ 18.0°C |
| Concentration UV (260 nm) | 287.3 μg/ml |
| Volume of eluted material | 738.4 ml |
| Step recovery (lysis and IEX) | 212 mg (134 %) |
| Concentration by TFF | |
| Load volume | 848.5 ml |
| Final concentrate volume | 200.8 ml |
| Concentration UV (260 nm) | 385.5 μg/ml |
| Step recovery | 77.4 mg (36.5 %) |
| Ion pair reverse phase chromatography | |
| Perfluorosorb-S-packed bed volume | 850 ml |
| Column cross sectional area | 28.27 $cm^2$ |
| Load volume | 200.8 ml |
| Volume of eluted material | 731.0 ml |
| Concentration of eluted pDNA | 79.4 μg/ml |
| Step recovery | 58.0 mg (74.9 %) |
| Formulation :10 mM Tris-hydrochloride, 1 mM EDTA 0.9 % (w/v) NaCl, pH 7.5 | |
| Final quantity of pDNA purified | 84.0 mg |
| Additional pDNA from batch 1 | About 27.3 mg |
| Final concentration of the pDNA purified | 1 908.0 μg/ml |
| Step recovery | 97.8 % |
| **Overall process recovery** | **35.9 %** |

**Example 2: *in vitro* study of pORT-RDD plasmid**

The aim of this study was to validate the new pORT-RDD plasmid *in vitro* after transfection: evaluation of RDD secretion efficacy and anti-proliferative activity. pORT-RDD plasmid was compared with a previous pVAX-RDD construct used as an internal positive control.

• Plasmids

*pORT plasmids*

pORT-RDD and pORT1aCMV (which was used as empty control plasmid) vectors are produced in the host strain DH1-ORT grown in LB media as described in Example 1. Cells are lysed and plasmid DNA are purified by ion-exchange chromatography, concentrated by tangential flow filtration, submitted to ion pair reverse phase chromatography and concentrated again by tangential flow filtration. The plasmids are formulated in 10 mM Tris, 1mM EDTA, 0.9% NaCl, pH 7.5, and kept frozen at -20°C.

*pVAXplasmids*

pVAX vectors were used as controls. pVAX1 (Invitrogen, ref V260-20) is a 3.0 kb plasmid vector which allows high-level transient expression of the protein of interest in most mammalian cells. The vector contains the following elements (map in Figure 3) : human CMV promoter, bovine growth hormone (BGH) polyadenylation signal and kanamycine resistance gene for selection in *E. coli.*

The RDD cassette, containing the human RDD gene under the control of the murine urokinase secretion signal, was cloned in pVAX1 between the CMV and the BGH polyA, to generate the pVAX-RDD.

pVAX1 and pVAX-RDD are produced by overnight bacteria culture in LB Broth medium 1500 ml + 50μg/ml kanamycin. Plasmids are purified with Nucleobond PC200EF kit (Macherey Nagel) and formulated in 10mM Tris, 1 mM EDTA, 0.9% NaCl, pH 7.5 and kept frozen at -20°C.

• Cell lines and culture conditions

Human C9 melanoma cells were grown in Dulbecco's Modified Eagles'Medium (DMEM + GlutaMAX, Invitrogen Gibco, ref 61965-026) supplemented with 10% heat inactivated foetal bovine serum (Invitrogen Gibco, ref 10270-106), at 37°C in a humidified 5% $CO_2$ atmosphere.

B16F10 murine melanoma cells (ATCC CRL-6475) were grown in Dulbecco's Modified Eagles' Medium + GlutaMAX supplemented with 10% heat inactivated foetal bovine serum, 1.5 g/l sodium bicarbonate (Invitrogen Gibco, ref 25080-060).

Vials of frozen cells were produced from a single vial, and maintained into liquid nitrogen.

Cells were subcultured twice a week (dilution 1:10 for C9 cells and 1:15 for B16F10 cells). For subculturing, medium is removed, rinsed with PBS, and 2 ml (for a 75 cm2 flask) of trypsin-EDTA solution (Invitrogen Gibco, ref 25300-096, lot 3107779) were added. The flask is allowed to sit at room temperature until the cells detach fresh medium is added, centrifuged at 1200 rpm for 5 min, aspirated, fresh medium is added, and the cells dispensed into new flasks.

• Transfection and proliferation assay

The day before the transfection, 60,000 C9 or 15,000 B16F10 cells / well (in 500 μl complete medium) were plated in 24-well plates. 1 μg of each plasmid (pVAX1, pVAX-RDD, pORT1aCMV, pORT-RDD) was transfected with the Lipofectamine Plus reagent (Gibco Invitrogen). Six wells / plasmid were prepared, and the experiment was performed twice.

The transfection protocol was adapted from the manufacturer procedures : 1 μg of plasmid DNA is incubated for 15 min at room temperature in 44 μl of Medium 0% and 6 μl of PLUS Reagent. Then 46 μl of Medium 0% and 4 μl of Lipofectamine are added and the mixture is incubated for 15 min at room temperature. 400 μl of Medium 0% and 100 μl of the ADN-Lipo-PLUS mix are mixed. Cells are washed with PBS and 500 μl of transfection mix are added per well. After 4 hours incubation at 37°C, the transfection medium is removed and 750 μl of complete medium are added. Transfected cells were incubated at 37°C for 96 hours.

Cells are incubated with 250 μl of PBS and 25 μl of MTT (Sigma) at 5 mg/ml. After 2 hr incubation at 37°C, 250 μl of lysis buffer (20% SDS-33% DiMethylFormamide-2% acetic acid-0.025N HCl-0.05N NaOH) are added overnight. 200 μl of each sample are distributed in 96-well plates for an optical density reading at 570 nm.

This experiment was repeated twice in the same conditions.

• DNA precipitation for resuspension buffer modification

Table 2 : Mixture for DNA precipitation

| | pORT1aCMV (2.2mg/ml) | pORT-RDD (1.96mg/ml) |
|---|---|---|
| Plasmid volume (μl) | 11.4 | 12.8 |
| Water (μl) | 188.6 | 187.2 |
| Sodium acetate 3M (μl) | 20 | 20 |
| 100% ethanol | 500 | 500 |

Two tubes of the above mixture are prepared and incubated overnight at -20°C. The mixture is centrifugated 30 min at 12 000rpm, 4°C. The supernatant is discarded, ethanol 70% is added. The solution is centrifuged, the supernatant discarded. The pellet is dried and then resuspended with 25μl of sterile NaCl 0.9% (one tube) or 25μl of sterile 10mM Tris - 1 mM EDTA -NaCl 0.9%, pH7.5 (called TE-NaCl) (the other tube).

DNA concentration is measured by optical density reading at 260/280 nm.

pORT1 aCMV- NaCl, pORT1aCMV - TENaCl, pORT-RDD - NaCl, and pORT-RDD - TENaCl DNA were transfected according to the previous transfection protocol, with 6 wells/plasmid. This experiment was performed once.

• RDD Western Blot

Human C9 melanoma cells were plated in 24-well plates (60,000 cells/well), and transfected with 1 μg of plasmid with Lipofectamine Plus Reagent (Gibco Life technologies), according to a protocol designed for proliferation assay. After 4 hours of transfection in serum-free medium, transfection supernatants were replaced by medium supplemented with10% serum.

Supernatants were collected 72h after transfection. For the cell extracts, cells were collected by scraping, and incubated for 30 min with 50 μl of lysis buffer (10 ml RIPA buffer + 1 antiprotease Complete mini tablet [ref. 11 836 153, Roche]). After centrifugation 10 min at 10 000 rpm, lysates were aliquoted and stored at -80°C.

Protein concentration for each supernatant was determined by the Bradford assay.

Western blot was performed by mixing 18 μl supernatant or 30 μg of total protein in 18 μl PBS for the extracts with 6 μl NuPage LDS sample buffer 4X (NP0007, Invitrogen) and 2.5 μl NuPage sample reducing agent (NP0004, Invitrogen). Samples are heated for 10 min at 70°C.

Samples are loaded on NuPage Novex Bis-Tris 12% gel (NP0341BOX, Invitrogen) according to NuPage Novex protocol and migration on MOPS SBS buffer.

A semi-dry transfer is carried out on nitrocellulose membrane Optitran BA-S 85 0,22μ (Schleicher & Schuell) with transfer buffer: Tris 48mM - glycin 39 mM - SDS 0.037% - methanol 20%, followed by blocking in PBS - 5% milk (blotting grade blocker non-fat dry milk, ref 170-6404, BioRad), overnight at 4°C. The membrane is washed PBS - 0.1 % Tween 20 and incubated with a primary antibody, Neosystem antiserum, diluted to 1:2000 in PBS - 0.1 % Tween 20 - 2% milk, 1 h30 at room temperature. After washing, the membrane is incubated with HRP donkey anti-rabbit IgG (NA934, Amersham), diluted to 1:5000 in PBS - 0.1 % Tween 20 - 2% milk, 1 h at room temperature. The membrane is washed and revealed with ECL (RPN2209, Amersham).

• Statistical analysis

All statistical analyses were performed using SigmaStat 3.1 software. The Student t test was used to compare the percentages of surviving cells after transfection with control plasmid or RDD plasmid. A p value $<0.05$ was considered significant.

•RESULTS

*RDD secretion driven by pORT-ROD plasmid*

A band was specifically detected at the expected size (10kDa) in both cell extracts and supernatants of pORT-RDD transfected cells.

However, RDD peptide was not detected in supernatant of pVAX-RDD transfected cells.

Thus, the complete CMV-intronA promoter from pORT vectors induced a stronger production and secretion of AMEP peptide than the classical CMV promoter from pVAX vectors.

*C9 proliferation inhibition after pORT-ROD transfection*

In order to demonstrate the antiproliferative activity of RDD peptide produced from pORT-RDD, a proliferation assay was set up after transfection of the cells. The new pORT-RDD plasmid was also compared to pVAX-RDD, a constitutive plasmid used as a positive control. The results of this experiment are shown in Table 3.

Table 3 : comparison of C9 antiproliferative activity of RDD bearing plasmid with control plasmid

| | Mean % surviving cells: RDD plasmid/ plasmid control | SD | % inhibition /control plasmid |
|---|---|---|---|
| pVAX1 | 100.0 | 12.2 | -22.2 |
| pVAX-RDD | 122.2 | 21.5 | |
| pORT1aCMV | 100.0 | 11.0 | 65.5 |
| pORT-RDD | 34.5 | 6.9 | |

pORT-RDD transfection significantly inhibit C9 melanoma cell proliferation by 65.5% versus pORT1aCMV control plasmid transfection (Student t test p<0.001). This inhibition was very reproducible as the same result was obtained for both experiments. In same conditions, pVAX-RDD plasmid transfection was unable to inhibit C9 proliferation.

*Effect of the DNA resuspension buffer*

The pORT-RDD plasmid is suspended in 10 mM Tris - 1mM EDTA - 150 mM NaCl, pH 7.5 (TENaCl). In order to know if the EDTA may interfere in the RDD activity, the antiproliferative activity of pORT-RDD plasmid prepared either in TENaCl or in physiologic serum (NaCl 0.9%) was assayed.

Table 4 : C9 proliferation assay with two DNA resuspension buffer

| | Mean | SD | % surviving cells /pORT |
|---|---|---|---|
| pORT, TENaCl | 0.247 | 0.019 | 100 |
| pORT-RDD, TENaCl | 0.041 | 0.006 | 16.5 |
| pORT, NaCl | 0.127 | 0.022 | 100 |
| pORT-RDD, NaCl | 0.036 | 0.007 | 28.3 |

A strong proliferation inhibition of C9 cells was obtained whatever the resuspension buffer. The difference did not reach statistical significance (p=0.291, Student t test).

This indicates that neither the Tris nor the EDTA interfere in the AMEP activity.

*B16F10 proliferation inhibition after pORT-RDD transfection*

In order to complete the antiproliferative activity of pORT-RDD on melanoma cells, murine B16F10 melanoma cells were transfected with the pORT1aCMV based plasmids and the MTT proliferation assay was performed.

Table 5 : B16F10 proliferation assays (mean of two experiments)

| | Mean % surviving cells / pORT1aCMV | SD | % inhibition |
|---|---|---|---|
| pORT1aCMV | 100.0 | 15.8 | 74.5 |
| pORT-RDD | 25.5 | 8.6 | |

pORT-RDD transfection significantly inhibit B16F10 cell proliferation by 74.5% versus pORT1aCMV control plasmid transfection (Student t test p<0.001).

Taken together, these results demonstrate that the pORT-RDD plasmid was more potent than the classical pVAX-RDD plasmid to induce a strong production and secretion of the AMEP peptide, and to inhibit the proliferation of melanoma cells (human C9 and murine B16F10). Furthermore, this anti-proliferative effect is independent of the resuspension buffer used to prepare the DNA. The EDTA contained in the TENacl buffer did not interfere in this activity.

**Example 3: study of antitumoral efficacy of a single pORT-RDD intratumoral electrotransfer in subcutaneous B16F10 melanoma tumors**

The aim of this study was to investigate the efficacy of a single curative intratumoral electrotransfer of the new pORT-RDD plasmid in pre-established subcutaneous B16F10 tumors, compared to empty control pORT1aCMV vector.

The effect on tumor growth was assessed by tumor volume monitoring.

• MATERIALS AND METHODS

*Cell line and culture conditions*

B16F10 melanoma cells were grown in Dulbecco's Modified Eagles'Medium + GlutaMAX supplemented with 10% heat inactivated foetal bovine serum, 1.5 g/l sodium bicarbonate and antibiotics (100 Ul/ml penicillin and 100 μg/ml streptomycin; Invitrogen Gibco, ref 15140-122) at 37 °C in a humidified 5% $CO_2$ atmosphere. Vials of frozen cells were produced from a single vial, and maintained into liquid nitrogen. Cells were subcultured twice or third a week depending on the confluence ratio as described in Example 2.

*Animals*

Female 6-8 week-old C57BU6 mice were provided by Janvier (Le Genest-St-Isle, France). All animal experiments were performed according to ethical guidelines for animal experimentation (Directive n°86/609 CEE) and were approved by BioAlliance Pharma ethical committee.
Animals were acclimatized for at least 4 days before tumor cell implantation, in the area where the experiment took place. The animals were maintained in rooms under controlled conditions of temperature (21 °C), photoperiod (12 h light/12 h dark) and air exchange (12 air renewals per hour). The humidity is kept between 30-70%. Animals were maintained in Specific Pathogen Free conditions, the room temperature and humidity were continuously monitored.

*Statistical tests*

All statistical analyses were performed using SigmaStat 3.1 software. To compare two groups, the software ran the Student t test when normality and equal variance tests were successfully passed. If one of these tests failed, the software ran a Mann&Whitney rank sum test. For each analysis, the user was free to accept each of the statistical tests. A p value <0.05 was considered significant.

• EXPERIMENTAL STUDY DESIGN AND TREATMENT

*Cell injection*

B16F10 cells were maintained in culture up to 5 passages before injection in mice. Cells were subcultured 48 h before the day of injection.
Subconfluent B16F10 cells were rinsed with PBS, and then incubated with trypsin-EDTA solution (Invitrogen Gibco, ref 25300-096) until cells detached. Fresh medium was added, cells were centrifuged at 1200 rpm for 5 min, and resuspended in 25 ml of fresh medium. Cells were counted in 0.04% Trypan Blue for viability quantification. Cells were centrifuged and resuspended in the adequate volume of 0.9% NaCl (Versol, Laboratoire Aguettant, Lyon, France) in order to have 106 B16F10 cells in 100 μl (cell mortality was higher in PBS buffer).
100 μl of cells were injected by SC route on the right flank of mice. Flanks of mice were previously shaved with an electric razor the day before the cell injection.

*Plasmid electrotransfer (day 0)*

Tumor volumes were first checked seven days after cell inoculation. At this moment, some tumors were already well established and then used in these first groups. In order to limit at best tumor volume variability at day 0, electrotransfer was performed on a first group of 14 mice (7 for pORT1aCMV and 7 for pORT-RDD), and two days later on a second group of 7 mice (3 for pORT1aCMV and 4 for pORT-RDD).

*Plasmid dilution*

50 μg of plasmid were administered per tumor in 50 μl of sterile buffer.
The dilutions of the plasmids were performed as follows, in sterile condition with sterile buffer (10mM Tris, 1 mM EDTA, 0.9% NaCl, pH 7.5):

First group : pORT1 aCMV: 364 μl of pORT1 aCMV at 2.2 μg/μl, were diluted with 436 μl of TE-NaCl; pORT-RDD: 408 μl of pORT-RDD at 1.96 μg/μl were diluted with 392 μl of TE-NaCl.

Second group: pORT1aCMV: because of a small quantity of control plasmid, the dilution used for the first group was kept frozen at -20°C, and re-used for the second group injections; pORT-RDD: 204 μl of pORT-RDD at 1.96 μg/μl were diluted with 196 μl of TE-NaCl.

*Study design*

C57BL/6 female mice bearing subcutaneous B16F10 tumors were assigned the following group numbers:

Table 6 : groups of treated mice

| Group | Treatment | Dose (μg plasmid per tumor) | No of treatment | Route | No of animals per group |
|---|---|---|---|---|---|
| 1 | pORT1 aCMV | 50 | 1 | IT | 7 |
| 2 | pORT-RDD | 50 | 1 | IT | 7 |
| 3 | pORT1 aCMV | 50 | 1 | IT | 3 |
| 4 | pORT-RDD | 50 | 1 | IT | 4 |

Tumors were measured before anaesthesia.
The animals were anesthetized by intraperitoneal injection of 0.2 ml of a ketamine / xylazine mix containing: 0.5 ml xylazine 2% (Rompun®, Bayer), 2.0 ml ketamine 50 mg/ml (Ketalar; Panpharma, Fougères, France), 7.5 ml NaCl 0.9%.

*Electrotransfer*

The treatment consisted of a single intratumoral injection of 50 μg of plasmid in 50 μl of TE-NaCl buffer. This dose was chosen because it corresponds to the dose usually used in the lab and in electrotransfer publications.
Conductive gel was applied on the tumor. The injection was immediately followed by electric pulses application by use of two stainless steel plate electrodes placed 5 mm apart on the tumor, with the Cliniporator device according to the following protocol:

- HV = 1500 V/cm, 100 μs, 1 Hz, 1 pulse
- pause = 1 000 ms
- LV = 140 V/cm, 400 ms, 1 pulse.

*Tumor volume monitoring and study of tumor growth*

Tumor size was monitored by measuring two perpendicular diameters with a digital caliper, on day 0, 2, 5, 7, 9, 12, and 14 post-electrotransfers.
Tumor volume was calculated according to the formula : $(\text{length} + \text{width}/2)^3 \times \pi/6$. Mice were not weighed during the experiment.
Dead mice were recorded during the experiment.
Tumor growth curves have been established based on either the tumor volume in $mm^3$, or the V/VO ratio (i.e. Volume at day D / Volume at day 0).
Inhibition of tumor growth was calculated as follows:

$$\%\text{Inhibition} = 100 \times [1 - (\text{Tumor volume in treated group}/\text{Tumor volume in control group})].$$

• RESULTS

*Mortality*

Natural deaths were observed during the experiment (at day 7 : pORT1aCMV, cage-1; pORT-RDD, cage-2; and pORT-RDD, cage-3). This mortality was not specially linked to the treatment. Natural deaths are often observed in the B16F10 model.

*Tumor volume monitoring*

Tumor volumes for all the tumors

The results are summarized in Table 7 and shown in Figure 4.

Table 7 : Tumor volume monitoring for all the tumors in $mm^3$

| Days | | 0 | 2 | 5 | 7 | 9 | 12 | 14 |
|---|---|---|---|---|---|---|---|---|
| pORT1aCMV | Mean | 59.26 | 87.93 | 178.02 | 279.02 | 338.89 | 640.13 | 864.31 |
| | SD | 14.13 | 31.56 | 103.36 | 201.88 | 216.33 | 454.42 | 571.78 |
| pORT-RDD | Mean | 56.03 | 80.99 | 104.11 | 130.28 | 221.48 | 409.07 | 642.71 |
| | SD | 18.65 | 16.52 | 40.91 | 29.97 | 76.86 | 132.96 | 182.96 |
| **% inhibition** | | | **7.9** | **41.5** | **53.3** | **34.6** | **36.1** | **25.6** |
| SigmaStat Student p= | | 0.662 | 0.529 | 0.041 | 0.026 | 0.144 | 0.163 | 0.285 |

On the day of the treatment, pORT1aCMV treated tumors ranged from 40.19 $mm^3$ to 84.76 $mm^3$ (mean volume of 59.26 $\pm$ 14.13 $mm^3$), and pORT-RDD treated tumors ranged from 33.51 $mm^3$ to 89.51 $mm^3$ (mean volume of 56.03 $\pm$ 18.65 $mm^3$).

At day 7, tumor volumes reached 279.02 $\pm$ 201.88 $mm^3$ in the pORT1aCMV control group, and 130.28 $\pm$ 29.97 $mm^3$ in the pORT-RDD treated group. An inhibition of 53% was thus observed (Student t test p = 0.026).

At day 14, tumor volumes reached 864.31 $\pm$ 571.78 $mm^3$ in the pORT1aCMV control group, and 642.71 $\pm$ 182.96 $mm^3$ in the pORT-RDD treated group, corresponding to 26% of tumor growth inhibition (non significant, Student t test p = 0.285).

In previous studies, according to the same protocol, with tumors < 100$mm^3$ at day 0, pVAX-RDD and pBi-RDD plasmids were shown to inhibit the B16F10 tumor growth by 20% and 29% respectively 14 days after electrotransfer, but without any statistical significance. Seven days after electrotransfer, the inhibition was less than 19%.

*Tumor volumes for only the small tumors*

Result analysis was also performed after selection of small tumors on the day of the treatment. The results are summarized in Table 8 and shown in Figure 6.

Table 8 : Tumor volume monitoring, expressed in $mm^3$, for tumors with a volume $\leq$ 50 $mm^3$

| Days | | 0 | 2 | 5 | 7 | 9 | 12 | 14 |
|---|---|---|---|---|---|---|---|---|
| pORT1 aCMV | Mean | 50.19 | 69.05 | 144.07 | 219.74 | 308.59 | 698.47 | 971.85 |
| | SD | 7.63 | 25.22 | 89.06 | 174.49 | 212.44 | 527.21 | 648.95 |
| pORT-RDD | Mean | 48.05 | 75.61 | 81.00 | 121.42 | 199.61 | 363.18 | 590.63 |
| | SD | 11.49 | 11.02 | 20.76 | 29.81 | 51.59 | 107.45 | 155.21 |
| % inhibition | | | | **43.8** | **44.7** | **35.3** | **48.0** | **39.2** |
| SigmaStat Student p= | | 0.705 | 0.545 | 0.095 | 0.168 | 0.213 | 0.126 | 0.158 |

pORT1 aCMV treated tumors ranged from 40.19 $mm^3$ to 59.73 $mm^3$ (mean volume of 50.19 $\pm$ 7.63 $mm^3$), and pORT-RDD treated tumors ranged from 33.51 $mm^3$ to 65.45 $mm^3$ (mean volume of 48.05 $\pm$ 11.49 $mm^3$).

At day 7, tumor volumes reached 219.74 $\pm$ 174.49 $mm^3$ in the pORT1aCMV control group, and 121.42 $\pm$ 29.81 $mm^3$ in the pORT-RDD treated group.

At day 14, tumor volumes reached 971.85 $\pm$ 648.95 $mm^3$ in the pORT1 aCMV control group, and 590.63 $\pm$ 155.21 $mm^3$ in the pORT-RDD treated group.

pORT-RDD intratumoral electrotransfer inhibited the growth B16F10 melanoma tumors by 45% at day 7 and 39% at day 14 after electrotransfer. But this difference did not reach statistical significance.

*Ratio V/V0 for only the small tumors*

In order to reduce the impact of the variability of tumor volumes at day 0, results were expressed in ration VNO (volume at day D / volume at day 0).

The results are shown in Table 9 and Figure 7.

Table 9 : Ratio V/VO for tumors with a volume ≤ 50 $mm^3$

| Days | | 0 | 2 | 5 | 7 | 9 | 12 | 14 |
|---|---|---|---|---|---|---|---|---|
| pORT1aCMV | Mean | 1.00 | 1.40 | 3.06 | 4.69 | 7.03 | 15.32 | 20.95 |
| | SD | 0.00 | 0.57 | 1.73 | 3.08 | 3.35 | 8.86 | 11.87 |
| pORT-RDD | Mean | 1.00 | 1.62 | 1.78 | 2.55 | 4.19 | 7.66 | 12.54 |
| | SD | 0.00 | 0.26 | 0.70 | 0.39 | 0.69 | 1.79 | 3.01 |
| % inhibition | | | | **42.0** | **45.6** | **40.3** | **50.0** | **40.1** |
| SigmaStat Student p= | | | | 0.098 | 0.094 | **0.05** | **0.046** | 0.096 |

At day 7, tumor volume ratio reached 4.69 ± 3.08 $mm^3$ in the pORT1aCMV control group, and 2.55 ± 0.39 $mm^3$ in the pORT-RDD treated group.

At day 14, tumor volumes reached 20.95 ± 11.87 $mm^3$ in the pORT1 aCMV control group, and 12.54 ± 3.01 $mm^3$ in the pORT-RDD treated group.

As detailed in the raw data (Table 9), at least 40% of tumor growth inhibition was maintained from day 5 to day 14, with a maximum of 50% at day 12. Statistical significance was obtained at days 9 and 12.

Taken together, these results indicate that pORT-RDD plasmid appears to be more potent than previous plasmids, especially seven days after the transfer. This inhibitory effect was more important if tumors are small (33.51 $mm^3$ to 65.45 $mm^3$) on the day of the treatment.

pORT-RDD intratumoral electrotransfer inhibited the growth B16F10 tumors by 45% at day 7, and 39% at day 14 after electrotransfer. But this difference did not reach statistical significance. This last point may be explained by the reduced number of mice per group (pORT1aCMV n=6, and pORT-RDD n=7). Furthermore, the reduced standard deviation observed for the pORT-RDD treated group was likely to be correlated to the antitumor effect of the RDD factor.

Indeed, it is largely known that antiangiogenic antitumoral factors are more potent on small tumors. So, for further experiments, tumor volumes may have to be checked earlier than 7 seven days after inoculation in order to treat small and calibrated tumors.

Furthermore, high cell mortality was observed after preparation of B16F10 cells in physiologic serum. In order to limit this cell mortality, other resuspension buffers may be assayed such as serum-free medium.

However, from this first experiment, it can be concluded that the pORT-RDD plasmid intratumoral electrotransfer inhibits the tumor growth of B16F10 melanoma tumors pre-established onto C57BU6 mice, with a better efficacy than with pBi and pVAX based vectors.

**Example 4: dose ranging evaluation following a single intratumoral electrotransfer of pORT-RDD in subcutaneous B16F10 tumors**

The aim of this study was to investigate the antitumoral efficacy of increasing doses of pORT-RDD plasmid following a single intratumoral electrotransfer, compared to vehicle electroporation, in subcutaneous pre-established B16F10 melanoma tumors. For this purpose, tumor volumes were comprised between 30 and 50 $mm^3$, and six groups were treated: 1) vehicle (10mM Tris, 1mM EDTA, 0.9% NaCl, pH 7.5 sterile buffer), 2) 25 μg, 3) 50 μg, 4) 100 μg, 5) 200 μg, and 6) 400 μg of therapeutic pORT-RDD plasmid. Tumor volumes have been monitored at least 14 days after electroporation treatment.

• EXPERIMENTAL STUDY DESIGN AND TREATMENT

*Cell injection*

B16F10 cells were maintained in culture up to 6 passages before injection in mice. Cells were subcultured 48 h before the day of injection.

Subconfluent B16F10 cells were rinsed with PBS, and then incubated with trypsin-EDTA solution (Invitrogen Gibco, ref 25300-096) until cells detached. Fresh medium was added, cells were centrifuged at 1200 rpm for 5 min, and resuspended in 25 ml of fresh medium. Cells were counted in 0.04% Trypan Blue for viability quantification. Cells were centrifuged and resuspended in the adequate volume of 0.9% NaCl (Versol, Laboratoire Aguettant, Lyon, France) in order to have $10^6$ B16F10 cells in 100 µl.

100 µl of cells were injected by SC route on the right flank of mice. Flanks of mice were previously shaved with an electric razor the day before the cell injection.

*Plasmid electrotransfer*

Six days after cell inoculation, mice were randomly assigned to pORT1aCMV, pVAX or pORT-RDD groups.

Increasing doses of plasmid were injected in 50 µl : 25 - 50 - 100 - 200 - 400 pg, leading to plasmid preparation at increasing concentrations : 0.5 - 1 - 2 - 4 - 8 mg/ml.

*Study design*

The C57Bl/6 mice were selected for treatment when the tumors reached 30 to 50 $mm^3$. The treatment was then performed at 3 different days, ready mice were included at random in each treatment group :

Table 10 : groups of treated mice

| Group | Treatment (DNA concentration) | Dose (µg plasmid per tumor in 50µl) | No of treatment | No of animals per group |
|---|---|---|---|---|
| 1 | Vehicle | none | 1 | 9 |
| 2 | pORT-RDD (0.5 µg/µl) | 25 | 1 | 9 |
| 3 | pORT-RDD (1.0 µg/µl) | 50 | 1 | 9 |
| 4 | pORT-RDD (2.0 µg/µl) | 100 | 1 | 10 |
| 5 | pORT-RDD (4.0 µg/µl) | 200 | 1 | 9 |
| 6 | pORT-RDD (8.0 µg/µl) | 400 | 1 | 9 |

Tumors were measured before anaesthesia.

The animals were anesthetized by intraperitoneal injection of 0.2 ml of a ketamine / xylazine mix containing: 0.5 ml xylazine 2% (Rompun®, Bayer), 2.0 ml ketamine 50 mg/ml (Ketalar; Panpharma, Fougères, France), 7.5 ml NaCl 0.9%.

*Electrotransfer*

The treatment consisted of an intratumoral injection of increasing doses of plasmid in 50 µl of TE-NaCl buffer. A conductive gel was applied on the tumor. The injection was immediately followed by electric pulses application by use of two stainless steel plate electrodes placed 5 mm apart on the tumor, with the Cliniporator device according to the protocol (previously used with pVAX-RDD intratumoral experiment):

- HV = 1500 V/cm, 100 µs, 1 Hz, 1 pulse
- pause = 1 000 ms
- LV = 140 V/cm, 400 ms, 1 pulse.

*Animals monitoring and sacrifice*

Tumor volume monitoring

After electroporation treatment, tumor volumes were monitored by measuring two perpendicular diameters (longest and largest diameters) with a digital calliper, at days 0, 2, 5, 7, 9, 12, 14 after treatment.

Tumor volume was calculated according to the formula : $(\text{length} + \text{width}/2)^3 \times \pi/6$.

Tumor growth curves have been established based on the tumor volume in $mm^3$.

Inhibition of tumor growth was calculated as follows:

$$\%Inhibition=100 \times [1 - (\text{Tumor volume in treated group}/\text{Tumor volume in control group})].$$

Bod weight monitoring

The body weight of animals was recorded the day prior cell injection, then the same days as for tumor volume monitoring.

Natural death monitoring

Natural deaths during the experiment were recorded. During the course of the experiment, animals showing signs of suffering (cachexia, weakening, and difficulty to move) and having loss more than 20% of their initial body weight, were sacrificed by $CO_2$ inhalation. Mice were sacrificed at the end of the experiment (at day 14) by $CO_2$ inhalation.

*Statistical tests*

All statistical analyses were performed using SigmaStat 3.1 software. The software ran the Student t test to compare two groups, and the ANOVA test to compare all the groups. A p value <0.05 was considered significant.

• RESULTS

*Mortality*

Natural deaths were observed during the experiment, and animals showing signs of suffering were sacrified. At day 14, the survival was: 22.2% in the buffer group (7 deaths/sacrifices out of 9), 55.6% in the 25 μg group (4 deaths/sacrifices out of 9), 66.7% in the 50 μg group (3 deaths/sacrifices out of 9), 90% in the 100 μg group (1 death/sacrifice out of 10), 77.8% in the 200 μg group (2 deaths/sacrifices out of 9), 77.8% in the 400 μg group (2 deaths/sacrifices out of 9).

This mortality was linked to the tumor development. None treated- and low dose treated-tumors showed the highest mortality.

*Body weight change*

No significant difference was observed in body weight between the groups. The small decrease in the buffer group at day 12 could be explained by a loss of weight of one mouse which was then sacrificed.

*Comparison of tumor volume monitoring*

Two operators measured the two perpendicular diameters of each tumor, at each day of the monitoring. Very minor differences were observed between tumor volumes measured by each operator

*Effect of the treatment on tumor volumes*

Tumor volumes measurements from one operator were used for analysis. The results are shown in Figure 8.

A dose-dependent inhibition of the growth of the pORT-RDD treated tumors was observed in comparison with control tumors. The inhibition was maximum at day 7, especially for the 200 μg dose with 80.3%:

Table 11 : dose-dependent inhibition of the growth of the pORT-RDD treated tumors

| | Doses | Day 2 | Day 5 | Day 7 | Day 9 | Day 12 | Day 14 |
|---|---|---|---|---|---|---|---|
| **% inhibition** | 25μg | 11.8 | 41.7 | 47.4 | 36.3 | 21.7 | |
| | 50μg | 27.1 | 57.3 | 60.3 | 48.7 | 17.8 | |
| | 100μg | 24.6 | 68.1 | 71.7 | 67.8 | 48.8 | 24.4 |
| | 200μg | 22.5 | 69.3 | 80.3 | 76.0 | 53.8 | 41.9 |
| | 400μg | 24.8 | 69.1 | 73.0 | 66.3 | 42.0 | 3.6 |

After day 7, all tumors grew again, especially control, 25 and 50 μg treated tumors. The control tumor growth curve decreased at day 14 because of a too small number of mice : a high mortality was observed from day 12.

The statistical analysis (one way analysis of variance) revealed that the inhibition for pORT-RDD treated groups was highly significant at days 5, 7, and 9 ($p<0.001$).

The two-and-two comparison by the Student t test showed that there was no statistical significance between 100, 200, and 400 μg groups. At day 12, statistical significance was obtained after one way analysis of variance, because of the re-growth of tumors. But, Student t test analysis revealed that statistical significance was reached for comparison of 50 and 200 μg doses.

In the initial protocol performed for former studies, a single dose of 50 μg of pORT-RDD plasmid was administered. This led to at best 50% of tumor growth inhibition. It has been decided to evaluate the antitumoral effect of increasing doses of the plasmid. Taken together, the pORT-RDD plasmid intratumoral electrotransfer significantly inhibits the growth of B16F10 melanoma tumors pre-established onto C57BL/6 mice, in a dose-dependent manner.

The 200 μg dose appeared to be the most effective dose to treat a 50 $mm^3$ tumor, as we obtained the highest inhibition and the best significance versus buffer, 25 and 50 μg groups. This dose corresponds to 4 μg per $mm^3$ of tumor.

Furthermore, the treatment did not induce any toxic effects even at the highest dose (400 μg), as mortality and body weight were not affected.

**Example 5: evaluation of repeated intratumoral electrotransfers of pORT-RDD plasmid in subcutaneous B16F10 tumors**

The dose ranging experiment described in example 4 showed that a single intratumoral electrotransfer of 200 μg of pORT-RDD plasmid was the most efficient dose compared to 25, 50, 100 and 400 μg doses, with about 80% of growth inhibition of subcutaneous melanoma B16F10 tumors at day 7 post-electrotransfer ($p<0.001$). After this time, tumors grew again.

These results prompted to assess the benefit of the treatment repetition on tumor growth. Thus, the aim of this study was to investigate the antitumoral efficacy of a repeated treatment at days 0 and 7. The treatment consists in intratumoral electrotransfer of 200 μg of pORT-RDD plasmid, at days 0 and 7, in subcutaneous pre-established B16F10 melanoma tumors of volumes comprised between 30 and 50 $mm^3$ at the first treatment. Four groups of mice have been defined: 1) vehicle (10 mM Tris, 1 mM EDTA, 0.9% NaCl, pH 7.5 sterile buffer), 2) empty control plasmid pORT1aCMV, 3) pORT-RDD single injection, 4) pORT-RDD repeated injections. Tumor volumes are monitored at least 14 days after electroporation treatment.

• EXPERIMENTAL STUDY DESIGN AND TREATMENT

*Cell injection*

B16F10 cells were maintained in culture up to 5 passages before injection in mice. Cells were subcultured 48 h before the day of injection.

The day of cell inoculation, subconfluent B16F10 cells were rinsed with PBS, and then incubated with trypsin-EDTA solution (Invitrogen Gibco, ref 25300-096) until cells detached. Fresh medium was added, cells were centrifuged at 1200 rpm for 5 min, and resuspended in 25 ml of fresh medium. Cells were counted in 0.04% Trypan Blue for viability quantification. Cells were centrifuged and resuspended in the adequate volume of 0.9% NaCl (Versol, Laboratoire Aguettant, Lyon, France) in order to have $10^6$ B16F10 cells in 100 μl.

100 μl of cells were injected by SC route on the right flank of mice. Flanks of mice were previously shaved with an electric razor the day before the cell injection.

*Plasmid preparation*

200 μg of plasmids are injected in 50 μl, leading to plasmid preparation at 4.0 μg/μl.

*Study design*

The study involved 50 C57BL/6 female mice bearing a 30 to 50 $mm^3$ tumor on their right flank. Treatment allocation has been decided at random and is as follows:

Table 12 : groups of treated mice

| Group | Treatments Day 0 | Day 7 | Dose per injection (µg in 50µl) | No of treatment | No of animals per group |
|---|---|---|---|---|---|
| 1 | Vehicle | None* | none | 1 | 10 |
| 2 | pORT1aCMV | None* | 200 | 1 | 10 |
| 3 | pORT-RDD | pORT1aCMV | 200 | 2 | 10 |
| 4 | pORT-RDD | pORT-RDD | 200 | 2 | 10 |
| * The second treatment is not performed for control groups because of the too large size of the tumors. At day 7, it is no longer possible to insert the tumor between the plate electrodes. | | | | | |

*Electrotransfer protocol*

The animals are anesthetized by intraperitoneal injection of 0.2 ml of a ketamine / xylazine mix containing: 0.5 ml xylazine 2% (Rompun®, Bayer), 2.0 ml ketamine 50 mg/ml (Ketalar; Panpharma, Fougères, France), and 7.5 ml NaCl 0.9%.

The treatments at days 0 and 7 consisted of intratumoral injections of 200 µg of plasmid in 50 µl of TE-NaCl buffer. A conductive gel was applied on the tumor. The injection was immediately followed by electric pulses application by use of two stainless steel plate electrodes placed 5 mm apart on the tumor, with the Cliniporator device according to the protocol:

- HV = 1500 V/cm, 100 µs, 1 Hz, 1 pulse
- pause = 1 000 ms
- LV = 140 V/cm, 400 ms, 1 pulse

Day 0 corresponds to the first day of electrotransfer treatment.

*Animals monitoring and sacrifice*

Monitoring of tumor volume, body weight and natural death were performed as described in Example 4.

## Claims

1. A pORT plasmid containing a sequence encoding all or part of a disintegrin domain of a metargidin or a derivative thereof under the control of the strong cytomegalovirus (CMV) promoter, wherein the said sequence is inserted.

2. A plasmid according to claim 1 containing a sequence encoding disintegrin domain of metargidin (RDD), wherein said plasmid has the sequence shown in SEQ ID NO:2.

3. A host cell transformed with the plasmid according to claim 1 or 2.

4. The host cell according to claim 3, which is a *Escherichia coli* bacterium.

5. The host cell according to claim 3, which is a mammalian cell.

6. A method of producing pORT-RDD plasmid which comprises the steps consisting of culturing a host cell according to claim 4, and recovering the pORT-RDD plasmid from the cultured host cells.

7. The method according to claim 6 which further comprises purifying the pORT-RDD plasmid by the steps of : (a) alkaline lysis of cultured host cells; (b) mesh bag filtration; (c) anion exchange chromatography; (d) concentration; (e) ion-pair reverse phase and size exclusion chromatographies, and (f) filtration.

8. A method of purifying pORT-RDD plasmid from a host cell according to claim 3, which method comprises the steps of : (a) alkaline lysis of host cells; (b) mesh bag filtration; (c) anion exchange chromatography; (d) concentration; (e) ion-pair reverse phase and size exclusion chromatographies, and (f) filtration.

9. A method of *in vitro* or *in vivo* expressing RDD peptide in a mammalian cell, which method comprises transforming said mammalian cell with pORT-RDD plasmid, whereby the RDD peptide is expressed in the mammalian cell.

10. The method according to claim 9, wherein the mammalian cell is a tumor cell.

11. A pharmaceutical composition comprising the plasmid according to claim 1 or 2, together with a pharmaceutically acceptable carrier.

12. Use of a plasmid according to claim 1 or 2, for the preparation of a medicament intended for the treatment of tumor.

13. The use according to claim 12, wherein said medicament is intended for the prevention and/or treatment of metastatic tumor.

14. The use according to claim 12 or 13, wherein said medicament is contacted with tumor cells and the tumor is electrically stimulated as follows :

    - first with at least one pulse of a High Voltage (HV) field strength of between 200 and 2000 volts/cm
    - second with a single pulse of Low Voltage (LV) field strength of between 50 and 200 volts/cm and of duration of between 300 and 2000 ms.

15. The use according to claim 14, wherein said medicament is contacted with the tumor cells by intratumoral injection.

**16.** The use according to claim 14, wherein said medicament is contacted with the tumor cells by intramuscular injection.

**17.** The use according to any one of claims 14 to 16 wherein the HV and LV pulses are separated by lag comprised between 300 ms and 3000 s.

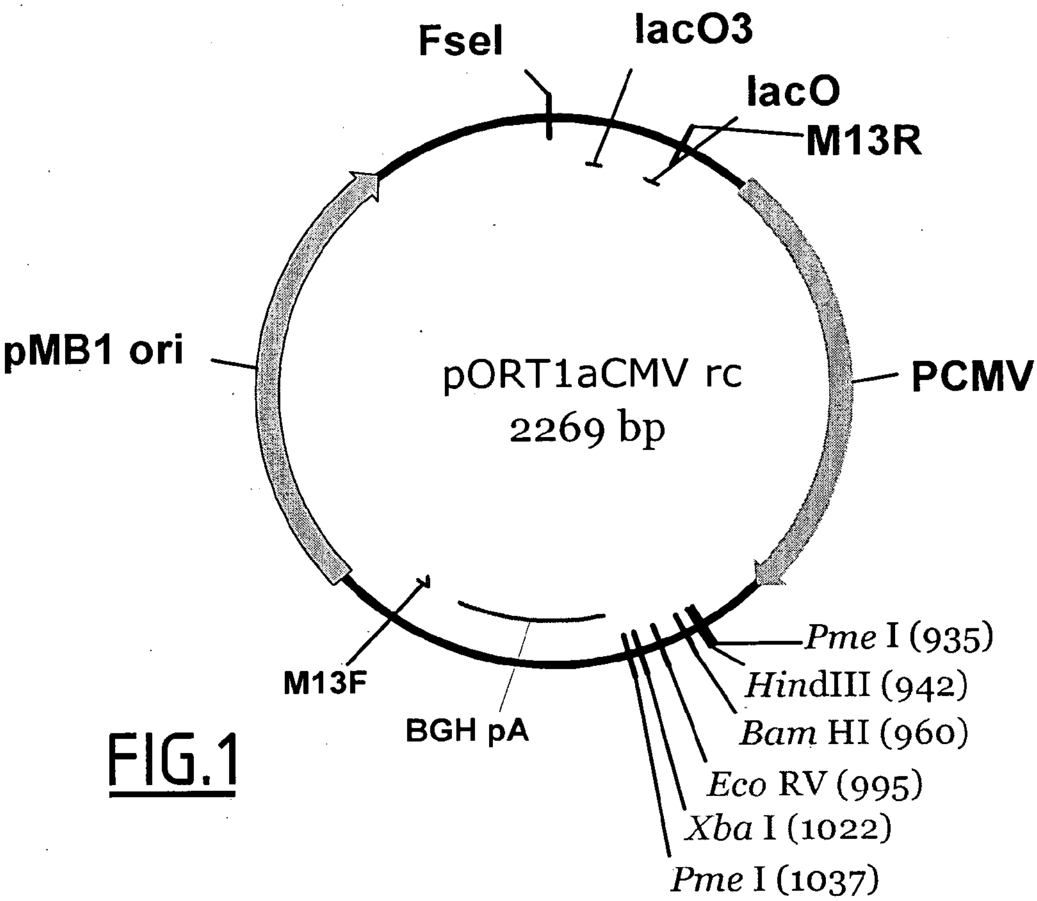
FseI
lacO3
lacO
M13R
pMB1 ori
pORT1aCMV rc
2269 bp
PCMV
Pme I (935)
HindIII (942)
Bam HI (960)
Eco RV (995)
Xba I (1022)
Pme I (1037)
M13F
BGH pA

FIG.1

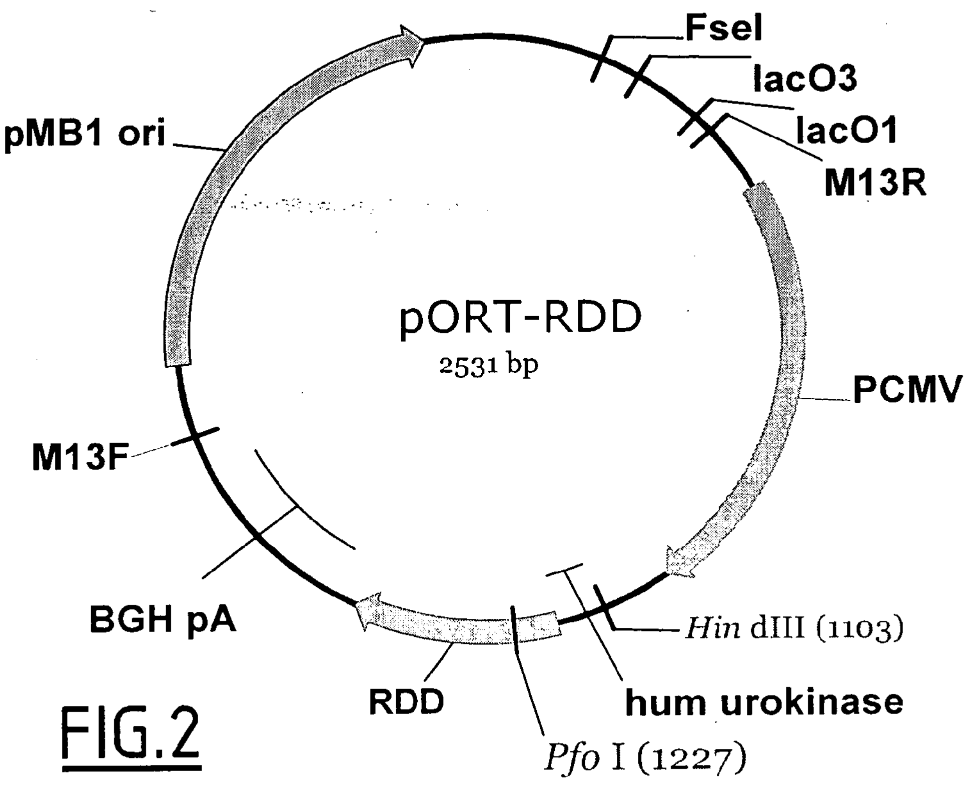
FseI
lacO3
lacO1
M13R
pMB1 ori
pORT-RDD
2531 bp
PCMV
M13F
BGH pA
Hin dIII (1103)
hum urokinase
RDD
Pfo I (1227)

FIG.2

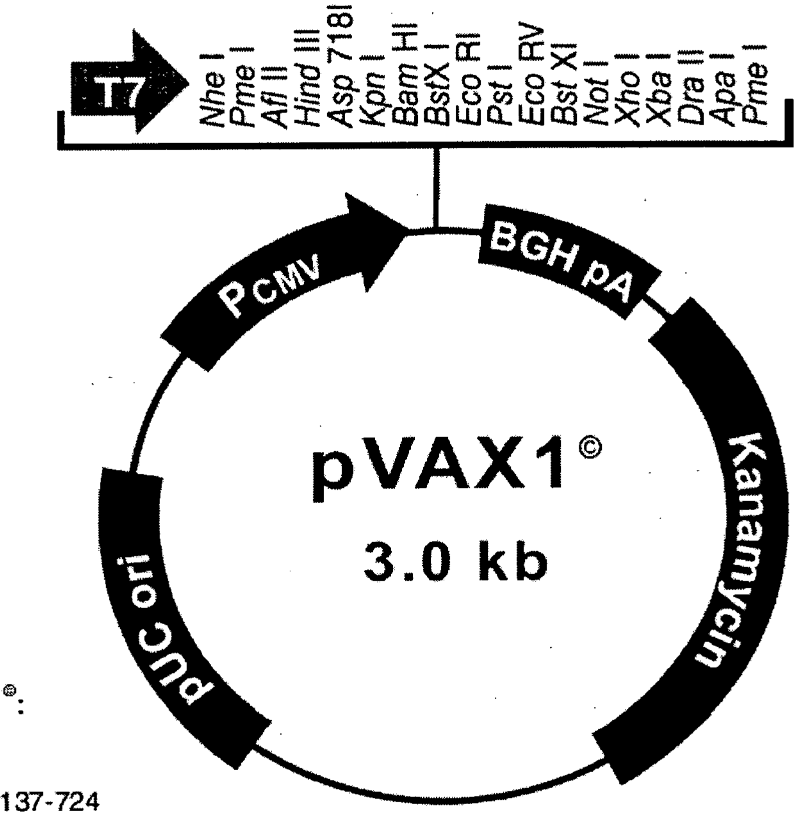


**Comments for pVAX1®:**
**2999 bp**

CMV promoter: bases 137-724
T7 promoter/priming site: bases 664-683
Multiple cloning site: bases 696-811
BGH reverse priming site: bases 823-840
BGH polyadenylation signal: bases 829-1053
Kanamycin resistance gene: bases 1226-2020
pUC origin: bases 2320-2993

FIG.3

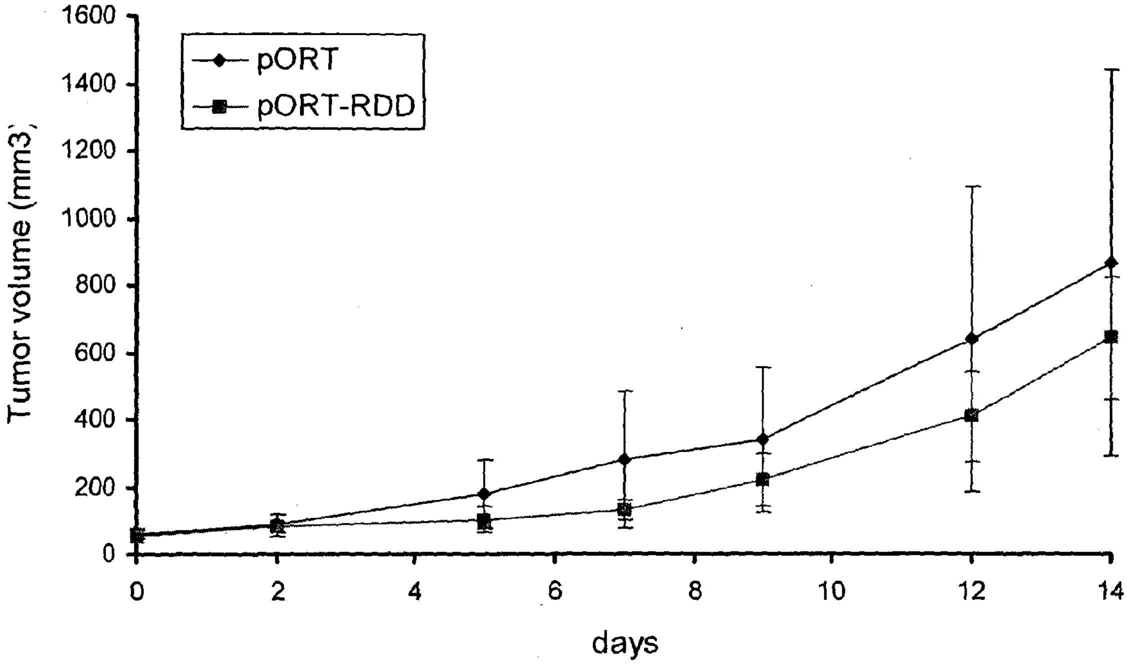
pORT
pORT-RDD
Tumor volume (mm3)
1600
1400
1200
1000
800
600
400
200
0
0
2
4
6
8
10
12
14
days

FIG.4

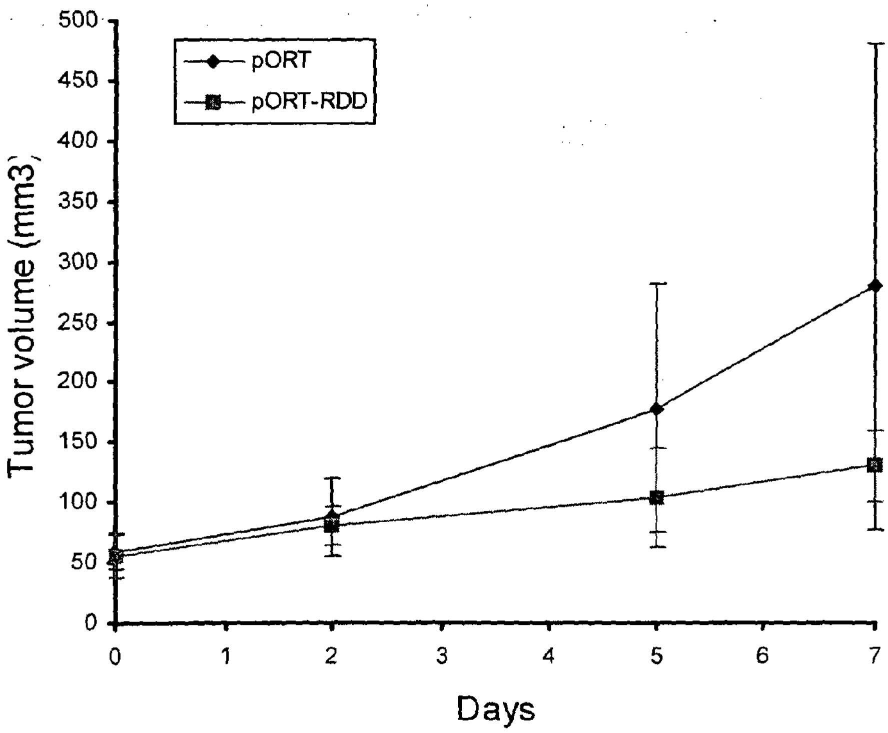
pORT
pORT-RDD
Tumor volume (mm3)
500
450
400
350
300
250
200
150
100
50
0
0
1
2
3
4
5
6
7
Days

FIG.5

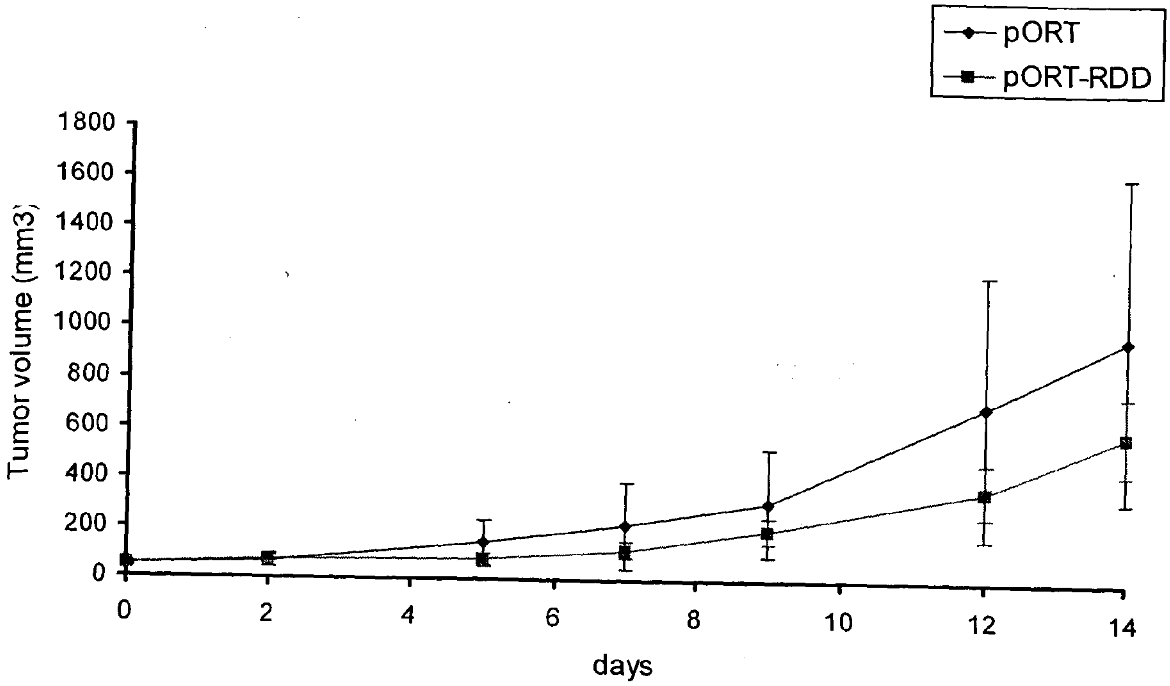
pORT
pORT-RDD
Tumor volume (mm3)
1800
1600
1400
1200
1000
800
600
400
200
0
0
2
4
6
8
10
12
14
days

FIG.6

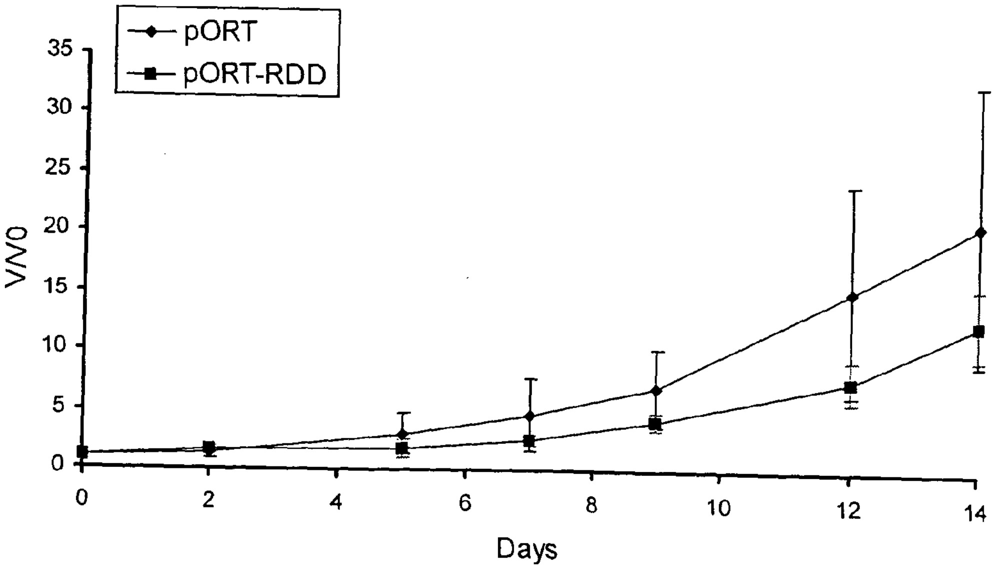
pORT
pORT-RDD
V/V0
35
30
25
20
15
10
5
0
0
2
4
6
8
10
12
14
Days

FIG.7

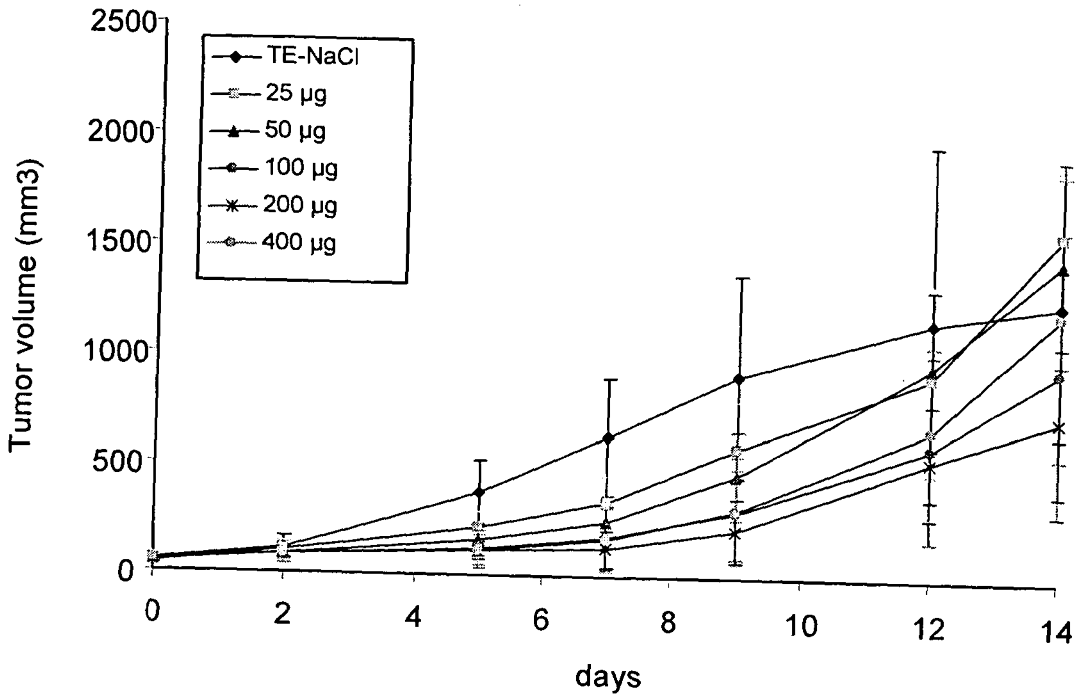

2500
2000
1500
1000
500
0
Tumor volume (mm3)
TE-NaCl
25 µg
50 µg
100 µg
200 µg
400 µg
0
2
4
6
8
10
12
14
days


FIG.8

European Patent Office

# PARTIAL EUROPEAN SEARCH REPORT

which under Rule 45 of the European Patent Convention shall be considered, for the purposes of subsequent proceedings, as the European search report

Application Number
EP 07 29 0285

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/009866 A (INST NAT SANTE RECH MED [FR]; TROCHON VERONIQUE [FR]; LU HE [FR]; SORI) 6 February 2003 (2003-02-06)<br>* page 15 *<br>* page 21 *<br>----- | 1-17 | INV.<br>C12N9/64 |
| Y | US 2005/176662 A1 (INANA GEORGE [US] ET AL) 11 August 2005 (2005-08-11)<br>* page 3; sequence 11 *<br>* page 16 - page 17 *<br>----- | 1-17 | |
| D,Y | ZHANG XI-PING ET AL: "Specific interaction of the recombinant disintegrin-like domain of MDC-15 (metargidin, ADAM-15) with integrin alphavbeta3"<br>JOURNAL OF BIOLOGICAL CHEMISTRY, AMERICAN SOCIETY OF BIOLOCHEMICAL BIOLOGISTS, BIRMINGHAM,, US,<br>vol. 273, no. 13,<br>27 March 1998 (1998-03-27), pages 7345-7350, XP002186268<br>ISSN: 0021-9258<br>* page 7345 *<br>-----<br>-/-- | 1-17 | TECHNICAL FIELDS SEARCHED (IPC)<br>C12N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Munich | 15 May 2007 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 07 29 0285

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| D,Y | KRAETZSCHMAR J ET AL: "METARGIDIN, A MEMBRANE-ANCHORED METALLOPROTEASE-DISINTEGRIN PROTEIN WITH AN RGD INTEGRIN BINDING SEQUENCE" JOURNAL OF BIOLOGICAL CHEMISTRY, AMERICAN SOCIETY OF BIOLOCHEMICAL BIOLOGISTS, BIRMINGHAM,, US, vol. 271, no. 9, 1 March 1996 (1996-03-01), pages 4593-4596, XP000644305 ISSN: 0021-9258 * page 4593 * | 1-17 | |
| Y | US 2006/177443 A1 (FANSLOW WILLIAM C III [US] ET AL) 10 August 2006 (2006-08-10) * page 2 * * page 6; example 1 * | 1-17 | TECHNICAL FIELDS SEARCHED (IPC) |
| Y,D | CRANENBURGH R M ET AL: "EFFECT OF PLASMID COPY NUMBER AND LAC OPERATOR SEQUENCE ON ANTIBIOTIC-FREE PLASMID SELECTION BY OPERATOR-REPRESSOR TITRATION IN ESCHERICHIA COLI" JOURNAL OF MOLECULAR MICROBIOLOGY AND BIOTECHNOLOGY, HORIZON SCIENTIFIC PRESS, WYMONDHAM,, GB, vol. 7, no. 4, 2004, pages 197-203, XP008046791 ISSN: 1464-1801 * page 197 - page 201 * | 1-17 | |
| | -/-- | | |

2

EPO FORM 1503 03.82 (P04C10)

European Patent Office

# PARTIAL EUROPEAN SEARCH REPORT

Application Number
EP 07 29 0285

| Category | Citation of document with indication, where appropriate, of relevant passages | Relevant to claim | CLASSIFICATION OF THE APPLICATION (IPC) |
|---|---|---|---|
| Y | DURANY OLGA ET AL: "Production of fuculose-1-phosphate aldolase using operator-repressor titration for plasmid maintenance in high cell density Escherichia coli fermentations" BIOTECHNOLOGY AND BIOENGINEERING, vol. 91, no. 4, August 2005 (2005-08), pages 460-467, XP002433752 ISSN: 0006-3592 * page 460 - page 461 * ----- | 1-17 | |

DOCUMENTS CONSIDERED TO BE RELEVANT

TECHNICAL FIELDS SEARCHED (IPC)

2

EPO FORM 1503 03.82 (P04C10)

European Patent Office

**INCOMPLETE SEARCH SHEET C**

Application Number
EP 07 29 0285

Although claims 9-10 are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 07 29 0285

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2007

| Patent document cited in search report | Publication date | Patent family member(s) | Publication date |
|---|---|---|---|
| WO 03009866 A | 06-02-2003 | CA 2453561 A1 | 06-02-2003 |
| | | CN 1535156 A | 06-10-2004 |
| | | EP 1409008 A1 | 21-04-2004 |
| | | FR 2827775 A1 | 31-01-2003 |
| | | JP 2005502638 T | 27-01-2005 |
| | | US 2004171549 A1 | 02-09-2004 |
| US 2005176662 A1 | 11-08-2005 | EP 1742641 A2 | 17-01-2007 |
| | | WO 2005077006 A2 | 25-08-2005 |
| US 2006177443 A1 | 10-08-2006 | US 2002042368 A1 | 11-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9709435 A


### Non-patent literature cited in the description


- **FOLKMAN.** *Nat Med,* 1995, vol. 1, 27-31
- **WOLFSBERG.** *J Cell Biol,* 1995, vol. 131, 275-8
- **KRÄTZSCHMAR et al.** *J Biol Chem,* 1996, vol. 271, 4593-6
- **HAM et al.** *Exp Cell Res,* 2002, vol. 279, 239-47
- **HERREN et al.** *FASEB J,* 1997, vol. 11, 173-80
- **MARTIN et al.** *J Biol Chem,* 2002, vol. 277, 33683-9
- **ZHANG et al.** *J Biol Chem,* 1998, vol. 273, 7345-50
- **NATH et al.** *Cell Sci,* 1999, vol. 112, 579-87
- **TROCHON-JOSEPH et al.** *Cancer Res,* 2004, vol. 64, 2062-2069
- **WILLIAMS et al.** *Nucleic Acids Res.,* 01 May 1998, vol. 26 (9), 2120-4
- **CRANENBURGH et al.** *Nucleic Acids Res.,* 01 March 2001, vol. 29 (5), E26
- **CRANENBURGH et al.** *J Mol Microbiol Biotechnol.,* 2004, vol. 7 (4), 197-203
- **VARLEY et al.** *Bioseparation,* 1999, vol. 8 (1-5), 209-17
- **DONG et al.** *Crit Rev Oncol Hematol.,* 21 December 2006
- Remington's Pharmaceutical Sciences. Mack Publishing Company, 1980
- **BIRNBOIM ; DOLY.** *Nucleic Acids Res.,* 24 November 1979, vol. 7 (6), 1513-23